# EUROPÄISCHE PATENTSCHRIFT

(11) **EP 0 783 515 B1**
(45) Veröffentlichungstag und Bekanntmachung des Hinweises auf die Patenterteilung: **24.08.2005**
(21) Anmeldenummer: 95932660.4
(22) Anmeldetag: 04.09.1995
(51) Int. Cl.: C07K 1/06, C07K 5/107, C07K 5/023, C07C 271/10, C07C 271/18

(54) **SCHUTZ- BZW. ANKERGRUPPEN UND DEREN VERWENDUNG (CARBAMIDE)**
PROTECTIVE AND LINKING GROUPS AND THEIR USE (CARBAMIDES)
GROUPES PROTECTEURS ET GROUPES DE LIAISON ET LEUR UTILISATION (CARBAMIDES)

(30) Priorität: 02.09.1994 DE 4431317
(43) Veröffentlichungstag der Anmeldung: 16.07.1997
(73) Patentinhaber: Gesellschaft für Biotechnologische Forschung mbH (GBF), 38124 Braunschweig (DE)
(72) Erfinder: HOFFMANN, Stefan, D-38124 Braunschweig (DE); FRANK, Ronald, D-38124 Braunschweig (DE)
(74) Vertreter: Boeters, Hans Dietrich
(86) Internationale Anmeldenummer: PCT/EP1995/003469
(87) Internationale Veröffentlichungsnummer: WO 1996/007672

(56) Entgegenhaltungen:
- US-A- 4 454 065
- KAWAI M. ET AL: 'Preparation of protected alpha- Methoxyglycine and Its Incorporation into Peptide Synthesis' CHEM.LETT. Bd. 90, Nr. 4, GB, Seiten 577 - 580, XP000565597
- BROWN R.A ET AL: 'Solid Phase Synthesis of alpha- Hydroxy Extended Peptides- Biological Precursurs of Peptide Amides' TETRAHEDRON LETT. Bd. 35, Nr. 5, GB, Seiten 789 - 92, XP000565606
- NICHIFOR M. ET AL: 'Synthesis of Peptide Derivatives of 5-Fluoracil' TETRAHEDRON Bd. 50, Nr. 12, GB, Seiten 3747 - 60, XP000565599

## Beschreibung

### Einleitung

Die regioselektive chemische Umsetzung einer Verbindung mit mehreren auch unterschiedlichen reaktiven chemischen Funktionen erfordert den Schutz aller dieser Funktionen bis auf diejenige(n), mit der (denen) eine chemische Reaktion eingegangen werden soll. Zum Schutz dieser Funktionen werden Molekülgruppen (Schutzgruppen) eingeführt. Diese lassen sich im Anschluß schonend und selektiv unter Zurückbildung der ursprünglichen Funktion entfernen. Für komplexe, mehrstufige Synthesen, insbesondere von Naturstoffen, wie Oligopeptide und Oligonucleotide, sind verschiedene Typen von Schutzgruppen notwendig. Sie zeichnen sich durch stark unterschiedliche Bedingungen der Abspaltung aus. Ein System von Schutzgruppen, in dem die einzelnen Typen so selektiv spaltbar sind, daß jeweils alle anderen Schutzgruppen unberührt bleiben, wird orthogonal genannt. Das Prinzip ist Gegenstand der Schutzgruppenchemie (siehe Protective Groups in Organic Synthesis, Greene, T. W. & Wuts, P. G. M. Eds., 2. Ed., 1991, John Wiley & Sons Inc., New York).

Weist der Schutzgruppentyp noch eine weitere reaktive Funktion-auf, so kann er kovalent und stabil mit einem Trägermaterial für die Festphasensynthese verknüpft werden. Es wird dann von einer "Ankergruppe" oder "Linkergruppe" gesprochen (siehe beispielsweise Breipohl et al. in *Tetrahedron Lett.* 28 (1987) 5651-5654 und Guibé et al. in *Tetrahedron Lett.* 30 (1989) 2641-2644).

Ein spezieller Typ von Schutzgruppe oder auch Ankergruppe ist derjenige, der erst durch eine vorgeschaltete chemische Reaktion in eine labile Form gebracht werden muß, die dann in einem zweiten Schritt unter sehr milden Bedingungen abgespalten werden kann (Geschützte Schutzgruppe - "Safety-Catch" Gruppierung; vgl. beispielsweise Patek in *Int. J. Peptide Protein Res.* 42 (1993) 97-117). Obwohl in einem solchen Fall zwei Reaktionsschritte zur Abspaltung notwendig sind, können deratige Gruppierungen große Vorteile haben:
(i) Eine derartige Gruppierüng kann sehr stabil gegen viele, auch sehr drastische Reaktionsbedingungen sein, aber durch die Folge von zwei spezifischen, sehr milden Reaktionsschritten gespalten werden.
(ii) Die labile Zwischenstufe der Schutzgruppe kann stabil genug sein, daß sie gute oder bessere Möglichkeiten zur Isolierung und Reinigung des Endproduktes bietet.

Aus US 4 454 065 sind nun folgende Verbindungen bekannt:
P-NH-CH(R₁)-CO-NH-CH(COQ)W
P-NH-CH(R₁)-CO-NH-CH(COQ)-X-R²
P-NH-CH(R₁)-CO-NH-CH(COQ)-O/S-R²
Bei R² handelt es sich um den Rest eines pharmazeutischen Wirkstoffs der Formel R²H.

Chemistry Letters, (1990) 577-580, 578 ist ferner eine Verbindung der folgenden Formel zu entnehmen:
Boc-L-Tyr(Bu^{t})-DL-Gly(Ome)-Ome
Boc-L-Tyr(Bu^{t})-DL-NH-CH(CO₂Me)-O-Me

Tetrahedron Letters, 35 (1994) 789-792, 790 beschreibt ferner eine Verbindung der Formel Fmoc-MH-CH(OCH₃)(CO₂H).

Tetrahedron, 50 (1994) 3747-3760, 3749 Schema 1 ist folgende Verbindung zu entnehmen:
H₂N-CH(R)-CO-NH-CH(CO₂Et)-FU
H₂N-CH(R)-CO-NH-CH(CO₂Et)-5-Fluoruracil.

Der Erfindung liegt die Aufgabe zugrunde, für eine Carbamidfunktion (CONH₂-Funktion) einen speziellen Typ von Schutz- bzw. Ankergruppe vorzuschlagen, der folgende Merkmale aufweist :
(i) die Schutz- bzw. Ankergruppe soll geschützt sein;
(ii) die labile Zwischenstufe soll unter geeigneten Reaktionsbedingungen stabil sein und eine Reinigung der Zwischenprodukte erlauben;
(iii) die labile Zwischenstufe soll in wäßriger physiologischer Pufferlösung, vorzugsweise bei neutralem pH (7) oder nahezu neutralem pH (5 bis 9) zerfallen können und die ursprüngliche Carbamidfunktion zurückbilden, so daß das Syntheseprodukt mit freier Carbamidfunktion direkt (ohne weitere Reinigung) in ein zellbiologisches oder biochemisches Testexperiment eingesetzt werden kann.

Erfindungsgemäß soll ferner für eine Carbamidfunktion (CO-NH₂-Funktion) ein spezieller Schutzgruppentyp als Ankergruppe für die Festphasensynthese von Peptiden (aber auch anderen Molekülstrukturen mit Carbamidfunktion) vorgesehen werden, insbesondere nach der Fmoc/tBu-Methode (Fmoc SPPS) (vgl. Fields & Noble in *Int. J. Peptide Protein Res.* 35 (1990) 161-214) und Boc/Bzl-Methode (Boc SPPS) (vgl. Barany et al. in Int. J. Peptide Protein Res. (1987) 705-739.

Gemäß einer Ausführungsform wird die der Erfindung zugrundeliegende Aufgabe durch ein Verfahren zur Herstellung eines durch eine temporäre Schutzgruppe geschützten Carbamids der allgemeinen Formel (I) gelöst:

R₁-CO-NH-C(R₂)(R₃)-X-Y (I)

worin
R₁-CO- einen Rest eines Naturstoffs als Produkt einer mehrstufigen Synthese bedeutet;
R₂ und R₃ Reste der Carbamid-Schutzgruppe bedeuten, die nicht an deren Funktion teilhaben, wobei R₂ und R₃ (i) stark elektronenziehende Gruppen gemäß der Erlenmeyer-Regel für O.O-, N.O- oder N.S-Acetale bedeuten und gleich oder verschieden sein können, oder (ii) verschieden sind, wenn einer der beiden Reste ein Wasserstoffatom bedeutet und der andere der beiden Reste eine stark elektronenziehende Gruppe gemäß der Erlenmeyer-Regel für 0.0-, N.O- oder N.S-Acetale ist;
X ein Sauerstoffatom oder ein Schwefelatom bedeutet und
Y eine Schutzgruppe für X bei einer Safety-Catch-Gruppierung bedeutet,
dadurch *gekennzeichnet,* daß man
(i) eine Verbindung der Formel

   H₂N-C(R₂)(R₃)-X-Y

   mit einer Verbindung der Formel

   R₁-COOH

   kondensierend umsetzt, wobei R₁-CO-, R₂, R₃, X und Y die vorstehend angegebenen Bedeutungen besitzen, und gegebenenfalls von der gebildeten Verbindung der Formel (I)
(ii) die Schutzgruppe Y abspaltet und das gebildete Zwischenprodukt (Zwischenstufe) bei einem pH außerhalb des Bereichs von pH 5 bis 9 stabilisiert und
(iii) danach im pH-Bereich von 5 bis 9 C(R₂)(R₃)=X freisetzt und das gebildete Zwischenprodukt in das freie Carbamid überführt.

Gemäß einer weiteren Ausführungsform wird die der Erfindung zugrundeliegende Aufgabe durch ein Verfahren zur Herstellung eines durch eine temporäre Schutzgruppe geschützen Carbamids der allgemeinen Formel I gelöst:

R₁-CO-NH-C(R₂)(R₃)-X-Y (I)

worin
R₁-CO- einen Rest eines Naturstoffs als Produkt einer mehrstufigen Synthese bedeutet;
R₂ und R₃ Reste der Carbamid-Schutzgruppe bedeuten, die nicht an deren Funktion teilhaben, wobei R₂ und R₃ (i) stark elektronenziehende Gruppen gemäß der Erlenmeyer-Regel für O.O-, N.O- oder N.S-Acetale bedeuten und gleich oder verschieden sein können, oder (ii) verschieden sind, wenn einer der beiden Reste ein Wasserstoffatom bedeutet und der andere der beiden Reste eine stark elektronenziehende Gruppe gemäß der Erlenmeyer-Regel für 0.0-, N.O- oder N.S-Acetale ist;
X ein Sauerstoffatom oder ein Schwefelatom bedeutet und
Y eine Schutzgruppe für X bei einer Safety-Catch-Gruppierung bedeutet, dadurch *gekennzeichnet,* daß man
   (i) (a) eine Verbindung der Formel C(R₂)(R₃)=X mit einer Verbindung der Formel R₁-CO-NH₂ zu einer Verbindung der Formel R₁-CO-NH-C(R₂)(R₃)-XH umsetzt und (b) die -XH-Gruppe des Reaktionsprodukts gemäß (a) in eine -X-Y-Gruppe überführt,
      wobei R₁, R₂, R₃, X und Y die vorstehend angegebenen Bedeutungen besitzen, und gegebenenfalls von der gebildeten Verbindung der Formel (I)
   (ii) die Schutzgruppe Y abspaltet und das gebildete Zwischenprodukt (Zwischenstufe) bei einem pH außerhalb des Bereichs von pH 5 bis 9 stabilisiert und
   (iii) danach im, pH-Bereich von 5 bis 9 C(R₂)(R₃)=X freisetzt und das gebildete Zwischenprodukt in das freie Carbamid überführt.

Die chemische Bindung zwischen Schutzgruppe und Carbamidfunktion kann also eine N-Acyl-N.O- bzw. N-Acyl-N.S-Acetalstruktur sein. Dieses N-Acyl-N.O-bzw. N-Acyl-N.S-Acetal kann durch Umsetzung der Carboxylfunktion mit der Aminofunktion eines geeigneten N.O- bzw. N.S-Acetals eingeführt werden, wobei die Sauerstoff- bzw. Schwefelfunktion geschützt ist und die Aminofunktion frei ist (Reaktionsweg A, vgl. graphische Darstellung). Sie kann ferner durch Umsetzung der Carbamidfunktion mit einer geeigneten Keto- oder Aldehydfunktion eingeführt werden (Reaktionsweg B, vgl. graphische Darstellung). Damit das labile N.O bzw. N.S-Acetal (II) mit freier Hydroxyl- bzw. Thiolfunktion isoliert werden kann, muß das N.O- bzw. N.S-Acetal von stark elektronenziehenden Substituenten flankiert sein. N.O- bzw. N.S-Acetale mit freier Hydroxyl- bzw. Thiolfunktion werden in wäßriger Lösung mehr oder weniger leicht unter Katalyse von Basen hydrolytisch gespalten. Die Hydroxyl- bzw. Thiolfunktion der Schutzgruppe soll daher durch eine weitere Schutzgruppe Y geschützt werden, welche die Hydrolyse des N.O- bzw. N.S-Acetals unter den Bedingungen der Synthese an R₁ verhindert. Die Gruppe Y soll unter den Bedingungen der Synthese an R₁ stabil sein.

### Prinzip

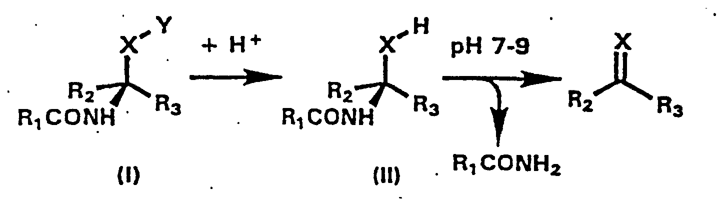

### Einführung der Schutzgruppe

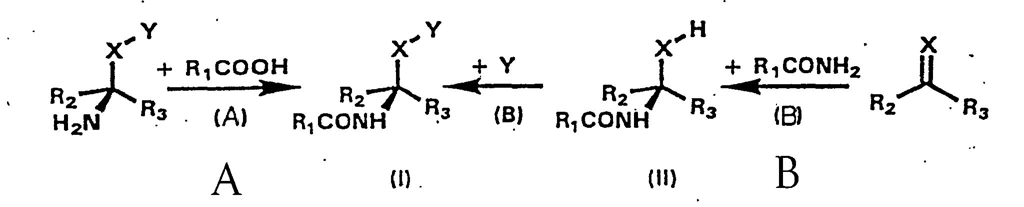

Bei der vorstehenden Darstellung des allgemeinen Prinzips der Schutz- bzw. Ankergruppe sowie des Synthesekonzeptes haben R₁,R₂,R₃ und Y die vorstehend angegebenen Bedeutungen.

Ein erfindungsgemäßes Verfahren kann dadurch gekennzeichnet sein, daß R₁-CO- eine Einheit für die Kette eines Peptids mit ein oder mehreren Aminosäureresten oder einen Carbonylrest eines Produkts einer mehrstufigen Peptidsynthese bedeutet.

Ferner kann ein erfindungsgemäßes Verfahren dadurch gekennzeichnet sein, daß R₂ und/oder R₃ eine Halogenalkylgruppe, beispielsweise eine Trifluormethylgruppe, oder eine ggf. derivatisierte Carboxylgruppe, beispielsweise eine -CO-βAla-OH-Gruppe, oder eine Alkylestercarboxylgruppe bedeuten, beispielsweise eine -COOCH₃-Gruppe.

Ferner kann ein erfindungsgemäßes Verfahren dadurch gekennzeichnet sein, daß R₂ oder R₃ eine zusätzliche reaktive Funktion zur Verknüpfung mit einem Trägermaterial aufweisen, beispielsweise eine Carboxyl-, eine Amino- oder eine Thiolgruppe.

Ferner kann ein erfindungsgemäßes Verfahren dadurch gekennzeichnet sein, daß die Schutzgruppe Y eine Alkylgruppe, beispielsweise eine Methyl-, Ethyl-, i-Propyl- oder t-Butylgruppe, eine substituierte Alkylgruppe, beispielsweise eine CH₃-O-CH₂- oder (CH₃)₃Si-CH₂-CH₂-O-CH₂-Gruppe, eine Arylgruppe oder eine Alkylsilylgruppe bedeutet, beispielsweise eine t-Butyldimethylsilylgruppe.

Ferner kann ein erfindungsgemäßes Verfahren dadurch gekennzeichnet sein, daß Y = CH₃ und R₂ = R₃ = CF₃ bedeuten.

Ferner kann ein erfindungsgemäßes Verfahren dadurch gekennzeichnet sein, daß man nach Stufe (i) und vor Stufe (ii) eine Peptidsynthese durchführt. Erfindungsgemäß kann die Peptidsynthese an einem Trägermaterial durchgeführt werden.

Schließlich wird die der Erfindung zugrundeliegende Aufgabe durch ein geschütztes Carbamid gelöst, das nach einem erfindungsgemäßen Verfahren erhältlich und dadurch gekennzeichnet ist, dass es mit einem Trägermaterial verknüft ist.

Nachstehend wird die Erfindung durch Beispiele näher erläutert.

### Experimenteller Teil (Allgemeine Methoden)

Es wurden folgende analytisch-spektroskopische Apparaturen verwendet.- ¹H-NMR/¹³C-NMR : Bruker Modell AM-300 und WM-400 mit Tetrametylsilan (TMS) als interner Standard.- ¹⁹F-NMR : H₃PO₄ als externer Standard.-Signalmultiplizität : s = Singulett, d = dublett, t = Triplett, q = Quartett, ⁿJ_{H,H} = magnetische Kopplung über n Bindungen zwischen benachbarten Protonen. Werden Signale diastereomerer Gemische voneinander getrennt registriert, so wird dies durch ein hochgestelltes [dia] deutlich gemacht.- FAB-MS : Kratos MS 50 TC RF mit neutralen Xenonstrahl (8-9 kV) und Finnigan Mat, Mass Spectrometer 8430 mit 3-Nitrobenzylalkohol als Matrix. Die Proben wurden in DMSO vorgelegt.- MALDI-TOF : Shimadzu Kratos Analytical Kompact MALDI III mit Sinapinsäure als Matrix.- UV/VIS : Carl Zeiss Modell PMQ II in Quarzküvetten mit 10 mm optischer Länge. ε(Dibenzofulven-Piperidin-Addukt/MeOH) = 5570. RP-C₁₈-HPLC : Analyt. HPLC : Pharmacia/ LKB Pump P 3500, Liquid Chromatography Controller LCC 500 Plus bzw. LKB 2249 Gradient Pump, LKB 2141 Variable Wavelength Monitor, Dreikanalflachbett-schreiber auf Machery-Nagel Nucleosil 300-7 C₁₈ 250x4.- Die schrittweise Synthese von Peptiden erfolgt nach den üblichen Methoden der Festphasen-peptidsynthese [Fields, G.B. and Noble, R.L., Int. J. Peptide Protein Res. **35**, 161-214 (1990)]. o-Chlorotritylharze (Novabiochem) werden nach den in der Literatur beschriebenen Verfahren beladen und die geschützten Peptide wie dort vom Harz abgespalten (Barlos, K; Chatzi, O.; Gatos, D. und Stavropoulos, G.; Int. J. Peptide Protein Res., **35** 161 (1990). Ankerbausteine sind mit (AB) und Modellverbindungen mit (MV) gekennzeichnet. Verbindungscodes : ([P] Schutzgruppe oder [L] Linkergruppe nach Weg [A] oder [B].[Beispiel].[Zahl]).

### Experimenteller Teil (Erläuterung anhand von Beispielen)

### Schutzgruppe für N^{α}-Fmoc/tBu-Festphasenpeptidsynthese

### Syntheseweg A / Beispiel 1

### 2-(9-Fmoc-amido)-2-methoxy-1.1.1.3.3.3-hexafluorpropan (PA.1.1)^{(MV)}

### Struktur

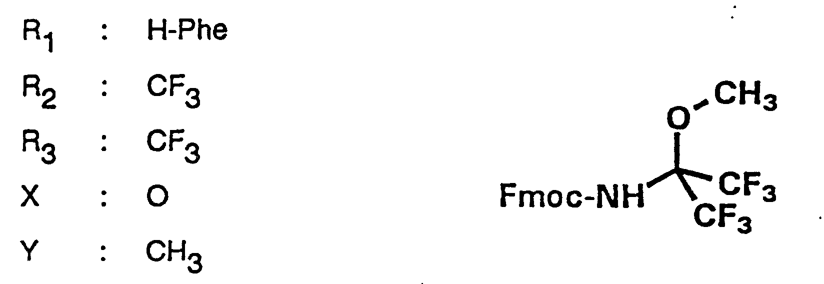

### Syntheseweg

### 2-(9-Fmoc-amido)-2-hydroxy-1.1.1.3.3.3-hexafluorpropan (PA.1.2)

### Summenformel (C₁₈H₁₃F₆NO₃)

120 mg (50 10⁻⁵ mol) Carbamidsäure-9-fluorenylmethylester werden in einer gesättigten Lösung von wasserfreien Hexafluoraceton (Vorsicht : giftig) (hergestellt durch langsames Eintropfen von Hexafluoraceton in ein Gemisch aus konz. Schwefelsäure und Phosphorpentoxid) in 5ml THF gelöst und 5 h bei RT gerührt. Das Reaktionsgemisch wird eingeengt, in 10ml Diethylether resuspendiert, filtriert und wieder eingeengt.- Ausbeute: 192 mg (95 % d. Th.) (weißer Feststoff).- ¹H-NMR (400 MHz, CDCl₃) : δ = 7.81(d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.62 (s (br.), 1H, NH), 4.6 (d, 2H, CH-CH₂), ³J_{H.H}= 6.67 Hz), 4.23 (t, 1H, CH-CH₂, ³J_{H,H}=6.67 Hz).- ¹³C-NMR (75 MHz, CDCl₃) : δ = 156.4 (s, NH-COO), 142.8 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 122.8 (s, CF₃), 120.3 (d, Fluorenyl-H₂), 119.0 (s, NH-COH), 68.1 (t, CH-CH₂), 46.8 (d, CH-CH₂).- ¹⁹F-NMR (376 MHz, CDCl₃) : δ = -82.2 (s, CF₃).- MS (FAB, 3-NBA) : m/z = 419 (15, [M+H]^{⊕}).

### 2-(9-Fmoc-amido)-2-methoxy-1.1.1.3.3.3-hexafluorpropan (PA.1.1)

### Summenformel (C₁₉H₁₅F₆NO₃)

101 mg (25 10⁻⁵ mol) (**PA.1.2**) werden in 4 ml abs. Methanol gelöst und 50 µl konz. Schwefelsäure zugesetzt. Es wird 12 h bei RT gerührt und das Reaktionsgemisch in eine gesättigte NaHCO₃-Lösung gegossen. Die organische Phase wird abgetrennt, 3 x mit gesättigter NaCl-Lösung extrahiert und über MgSO₄ getrocknet. Die organische Phase wird eingeengt und unter Petroleumbenzin kristallisiert.- Ausbeute: 192 mg (95 % d. Th.) (weißer Feststoff).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.62 (s (br.), 1H, NH), 4.6 (d, 2H, CH-CH₂), ³J_{H.H}= 6.67 Hz), 4.23 (t, 1 H, CH-CH₂, ³J_{H.H}= 6.67 Hz), 1.55 (s, 3H, CH₃).- ¹³C-NMR (75 MHz, CDCl₃) : δ = 156.4 (s, NH-COO), 142.8 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 122.8 (s, CF₃), 120.3 (d, Fluorenyl-H₂), 119.0 (s, NH-COH), 68.1 (t, CH-CH₂), 46.8 (d, CH-CH₂), 54.4 (q, CH₃).- ¹⁹F-NMR (376 MHz, CDCl₃) : δ = -82.2 (s, CF₃).- MS (FAB, 3-NBA) : m/z = 419 (15, [M+H]^{⊕}).

### Anwendung

(**PA.1.1**) wird mit 95% TFA/2.5% TIBS/2.5% Wasser entschützt und das Reaktionsprodukt chromatographisch isoliert. Das entschützte Produkt wird mit Puffersystem (a) / 30% Ethanol bei RT Carbamidsäure-9-fluorenylester hydrolysiert. Die Hydrolyse erfolgt bei RT innerhalb 15min.

### Syntheseweg B / Beispiel 2

### 2-(N^{α}-Ac-Phe-NH)-2-methoxy-1.1.1.3.3.3-hexafluorpropan (PB.2.1)^{(MV)}

### Struktur

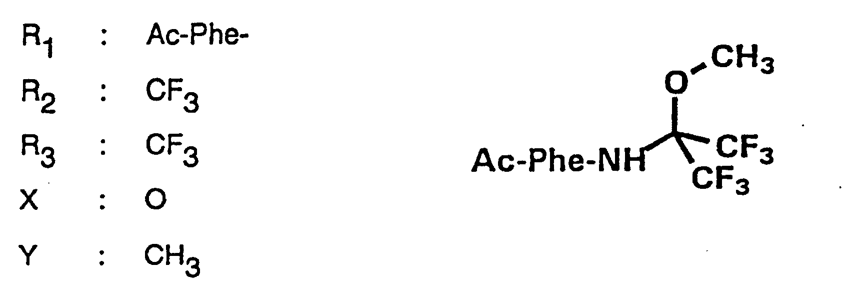

### Syntheseweg

### 2-(N^{α}-Ac-Phe-NH)-2-hydroxy-1.1.1.3.3.3-hexafluorpropan (PB.2.2)

### Summenformel (C₁₄H₁₄F₆N₂O₃)

103.1 mg (50 10⁻⁵ mol) N^{α}-Acetyl-phenylalanylamid werden analog zu **(PA.1.2**) umgesetzt. Das Reaktionsgemisch wird eingeengt und unter Petroleumbenzin kristallisiert.- Ausbeute: 182 mg (98% d. Th.) (weißer Feststoff).- ¹H-NMR (300 MHz, CDCl₃) : δ = 8.95 (s, 1H, NH), 8.60 (s, 1H, OH), 7.3-7.05 (m, 5H, Phenyl-H), 6.15 (d, 1H, CONH, ³J_{H.H}= 7.9Hz), 4.97 (AB-q, 1H, CH-NH, ³J_{H.H}= 7.9Hz, ³J_{H.H}=6.7Hz), 3.12 (AB-q, 1H, CH₂-C₆H₅, ³J_{H.H}= 6.7Hz, ²J_{H.H}= 14.0Hz), 3.12 (AB-q, 1H, CH₂-C₆H₅, ³J_{H.H}= 6.7Hz, ²J_{H.H}= 14.0Hz), 1.92 (s, 3H, CH₃).- ¹³C-NMR (75 MHz, CDCl₃) : δ = 177.5 (s, CH₃-CO), 170.5 (s, CO-NH), 135.1 (s, Phenyl-H), 129.2 (d, Phenyl-H), 128.9 (d, Phenyl-H), 127.5 (d, Phenyl-H), 127.5 (s, Phenyl-H), 120.5 (q, CF₃, ³J_{H.H}= 270Hz), 83.9 (m, NH-COH), 68.1 (t, CH-CH₂), 54.7 (d, NH-CH), 37.8 (t, CH₂-C₆H₅), 22.6 (q, CH₃).- ¹⁹F-NMR (376 MHz, CDCl₃) : δ = -82.1 (s, CF₃).- MS (FAB, 3-NBA) : m/z = 373 (15, [M+H]^{⊕}).

### 2-(N^{α}-Ac-Phe-NH)-α-methoxy-1.1.1.3.3.3-hexafluorpropan (PB.2.1)

### Summenformel (C₁₅H₁₆F₆NO₃)

93.1 mg (25 10⁻⁵ mol) (**PA.2.2**) werden analog zu (**PA.1.2**) in Methanol umgesetzt.- Ausbeute : 192 mg (95% d. Th.) (weißer Feststoff unter Petroleumbenzin).- ¹H-NMR (300 MHz, CDCl₃) : δ = 8.95 (s, 1H, NH), 8.60 (s,1H, OH), 7.3-7.05 (m, 5H, Phenyl-H), 6.15 (d, 1H, CONH, ³J_{H.H}= 7.9Hz), 4.97 (AB-q, 1H, CH-NH, ³J_{H.H}= 7.9Hz, ³J_{H.H}=6.7Hz), 3.12 (AB-q, 1H, CH₂-C₆H₅, ³J_{H.H}= 6.7Hz, ²J_{H.H}= 14.0Hz), 3.12 (AB-q, 1H, CH₂-C₆H₅, ³J_{H.H}= 6.7Hz, ²J_{H.H}= 14.0Hz), 1.92 (s, 3H, CH₃), 1.55 (s, 3H, CH₃).- ¹³C-NMR (75 MHz, CDCl₃) : δ = 177.2 (s, CH₃-CO), 171.5 (s, CO-NH), 135.1 (s, Phenyl-H), 129.2 (d, Phenyl-H), 128.9 (d, Phenyl-H), 127.5 (d, Phenyl-H), 127.5 (s, Phenyl-H), 120.5 (q, CF₃, ³J_{H.H}= 270Hz), 83.9 (m, NH-COH), 68.1 (t, CH-CH₂), 54.7 (d, NH-CH), 54.4 (q, CH₃), 37.8 (t, CH₂-C₆H₅), 22.6 (q, CH₃).-¹⁹F-NMR (376 MHz, CDCl₃) : δ = -82.1 (s, CF₃).- MS (FAB, 3-NBA) : m/z = 388(11, [M+H]^{⊕}).

### Anwendung

(**PB.2.1**) wird mit 95% TFA/2.5% TIBS/2.5% Wasser entschützt und das Reaktionsprodukt chromatographisch isoliert. Das entschützte Produkt wird mit Puffersystem (a) bei RT und 50°C zum N^{α}-Acetyl-phenylalanylamid hydrolysiert. Die Hydrolyse erfolgt bei RT innerhalb 15min und bei 50°C innerhalb von 5min.

### Syntheseweg B / Beispiel 3

### 2-(N^{α}-9-Fmoc-Asn-OMe)-2-(MeO)-1.1.1.3.3.3-hexafluorpropan(PB.3.1)^{(MV)}

### Struktur

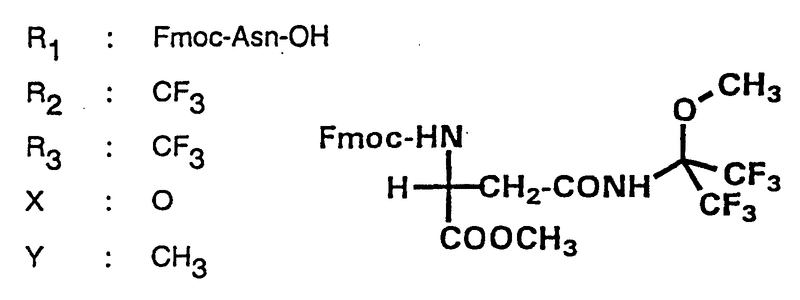

### Syntheseweg

### 2-(N^{α}-9-Fmoc-Asp-β-amido)-2-hydroxy-1.1.1.3.3.3-hexafluorpropan (PB.3.2)

### Summenformel (C₁₄H₁₄F₆N₂O₃)

103.1 mg (50 10⁻⁵ mol) N^{α}-9-Fmoc-asparagin werden analog zu (**PA.1.2**) umgesetzt. Das Reaktionsgemisch wird eingeengt und unter Petroleumbenzin kristallisiert.- Ausbeute: 182 mg (98% d. Th.) (weißer Feststoff).- ¹H-NMR (300 MHz, CDCl₃) : δ = 7.81(d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 6.05 (d, 1H, NH), 4.61 (s(br), 1H, NH-CH-CO), 4.23 (m, 3H, CH-CH₂/CH-CH₂).- ¹³C-NMR (100 MHz, CDCl₃) : δ = 172.8 (s, COOH), 168.5 (s, CO-NH), 156.0 (s, NH-COO), 142.8 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 122.8 (s, CF₃), 120.3 (d, Fluorenyl-H₂), 119.0 (s, NH-COH), 77.2 (d, NH-CH-CO), 68.1 (t, CH-CH₂), 46.8 (d, CH-CH₂), 38.8 (t, CH₂-CO).- ¹⁹F-NMR (376 MHz, CDCl₃) : δ = -81.8 (s, CF₃).- MS (FAB, 3-NBA) : m/z = 521 (15, [M+H]^{⊕}).

### 2-(N^{α}-9-Fmoc-Asn-OMe)-2-(methoxy)-1.1.1.3.3.3-hexafluorpropan (PB.3.1)^{(MV)}

### Summenformel (C₁₅H₁₆F₆NO₃)

93.1 mg (25 10⁻⁵ mol) (**PB.3.1**) werden analog zu (**PA.1.3**) in Methanol umgesetzt.- Ausbeute: 192 mg (95% d. Th.) (weißer Feststoff unter Petroleumbenzin).- ¹H-NMR (300 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 6.05 (d, 1H, NH), 4.61 (s(br), 1H, NH-CH-CO), 4.23 (m, 3H, CH-CH₂/CH-CH₂), 3.86 (s, 3H, COOCH₃), 3.42(s, 3H, CH₃O).- ¹³C-NMR (100 MHz, CDCl₃) : δ = 172.8 (s, COOH), 168.5 (s, CO-NH), 156.0 (s, NH-COO), 142.8 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 122.8 (s, CF₃), 120.3 (d, Fluorenyl-H₂), 119.0 (s, NH-COH), 77.2 (d, NH-CH-CO), 68.1 (t, CH-CH₂), 54.4 (q, CH₃), 52.6 (q, COOCH₃), 46.8 (d, CH-CH₂), 38.8 (t, CH₂-CO).- ¹⁹F-NMR (376 MHz, CDCl₃) : δ = -81.8 (s, CF₃).- MS (FAB, 3-NBA) : m/z = 551 (15, [M+H]^{⊕}).

### Anwendung

(**PA.3.1**) zeigt vollständige Stabilität gegenüber 20% Piperidin /DMF über 3h bei RT. (**PA.3.1**) wird mit 95% TFA/2.5% TIBS/2.5% Wasser entschützt und das Reaktionsprodukt chromatographisch isoliert. Das entschützte Produkt wird mit Puffersystem (a) / 40% Ethanol bei RT und 50°C zum Fmocasparaginmethylester hydrolysiert. Die Hydrolyse erfolgt bei RT innerhalb 15min und bei 50°C innerhalb von 5min.

### Ankerbaustein für die N^{α}-Fmoc/tBu-Festphasenpeptidsynthese

### Syntheseweg A / Beispiel 1

### N^{ε}-Boc-Lys-Phe-Phe-α-rac-tert-butoxy-gtycyl-βAla-OH (LA.1.1)^{(MV)}

### Struktur

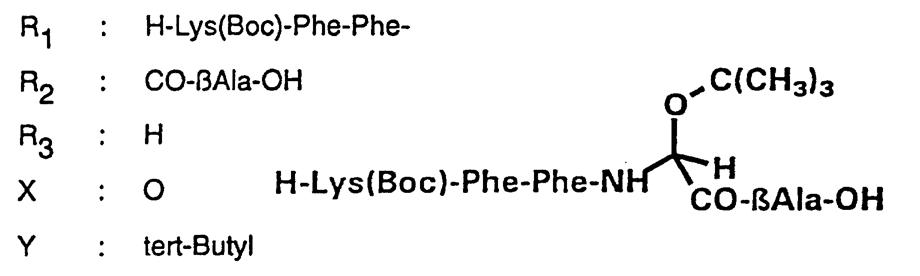

### Syntheseweg

### N^{α}-9-Fmoc-α-rac-hydroxy-glycin (LA.1.2)

### Summenformel (C₁₇H₁₅NO₅)

10.74 g (45 10⁻³ mol) Carbamidsäure-9-fluorenylmethylester [Carpino, L. A.; Mansuor, E. M. E.; Cheng, C. H.; Williams, J. R., MacDonald, R.; Knapczyk, J. und Carman, E., *J. Org. Chem*., 48 (1983) 661] werden zusammen mit 4.53 (50·10⁻³ mol) Glyoxalsäurehydrat in einem Gemisch von 50 ml DCM und 40 ml THF 2d bei RT gerührt. Die Lösung wird eingeengt und der Rückstand in Ethylacetat gelöst. Es wird zweimal mit je 150 ml Wasser extrahiert und die organische Phase über MgSO₄ getrocknet. Die organische Phase wird am Rotationsverdampfer eingeeengt und (**LA.1.2**) aus Ethylacetat/Toluol umkristallisiert.- Ausb. 12.55g (89% d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (d, 1H, NH), 5.47 (d, 1H, NH-CHOH), 4.4 (d, 2H, CH-CH₂), ³J_{H.H}= 6.67 Hz), 4.23 (t, 1H, CH-CH₂, ³J_{H.H}=6.67 Hz).- ¹³C-NMR (75 MHz, CDCl₃) : δ = 168.9 (s, COOH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.9 (d, NH-CHOH), 68.1 (t, CH-CH₂), 46.8 (d, CH-CH₂).- MS (FAB, 3-NBA) : m/z = 315 (23, [M+H]^{⊕}).

### N^{α}-9-Fmoc-α-rac-hydroxy-glycinbenzylester (LA.1.3)

### Summenformel (C₂₄H₂₁NO₅)

1.57 g (5 10⁻³ mol) (**LA.1.2**) und 815 mg (2.5 10⁻³ mol) Cäsiumcarbonat werden in 17.6 ml 80%igen wässrigen Ethanol suspendiert. Die Lösung wird bis vollständig eingeengt und wiederholt (3x) in 30 ml absoluten Ethanol resuspendiert und eingeengt. Der Rückstand wird kurz im HV getrocknet und in 15 ml DMF suspendiert. Es werden 627 µl (5 10⁻³ mol) Benzylbromid zugesetzt und 2d bei RT geschüttelt. Das Reaktionsgemisch wird in Eiswasser gegossen und die wässrige Phase mit Ethylacetat extrahiert. Die organische Phase wird mit gesättigtem NaHCO₃-, gesättigter NaCl, 0.1 M Salzsäure und gesättigter NaCl-Lösung gewaschen und über MgSO₄ getrocknet. Die organische Phase wird eingeengt und (**LA.1.3**) aus Dichlormethan/ Petroleumbenzin umkristallisiert- Ausbeute : 1.85 g (92% d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.95 (d, 1H, NH), 5.47 (d, 1H, NH-CHOH), 5.23 ('d', 2H COOCH₂), 4.4 (d, 2H, CH-CH₂), ³J_{H.H}= 6.67 Hz), 4.23 (t, 1H, CH-CH₂, ³J_{H.H}=6.67 Hz).- ¹³C-NMR (75 MHz, CDCl₃) : δ = 168.5 (s, COOH), 154.2 (s, NH-COO), 143.7 (s, Fluorenyl-C₆), 141.4 (s, Fluorenyl-C₅), 135.2 (s, Phenyl-H), 128.57/128.4/128.1/127.1/125.1/120.0 (d, Fluorenyl-C / Phenyl-C), 78.4 (d, NH-CHOH), 67.2 (t, COO-CH₂), 67.1 (t CH-CH₂), 47.1 (d, CH-CH₂).

### N^{α}-9-Fmoc-α-rac-tert-butoxy-glycinbenzylester (LA.1.4)

### Summenformel (C₂₈H₂₉NO₅)

**Methode 1 :** 100 mg (25 10⁻⁵ mol) (**LA.1.3**) werden in 500 µl absoluten Dioxan und 250 µl absoluten Diethylether in einer dickwandigen Glasampulle gelöst und 5 µl konz. Schwefelsäure zugesetzt. Es werden ca. 250 µl Isobuten bei - 45°C einkondensiert und die Ampulle verschlossen. Es wird 8h bei 4°C geschüttelt und das Reaktionsgemisch in 50 ml ges. NaHCO₃-Lösung eingegossen. Es wird 2x mit 100 ml Ethylacetat extrahiert und die organische Phase 2x mit je 100 ml gesättigter NaCl-, 10%iger Citronensäure-, gesättigter NaCl-Lösung gewaschen und über MgSO₄ getrocknet. (**LA.1.4**) wird durch RP-C₁₈-HPLC mit Wasser/Acetonitril isoliert.- Ausbeute : (30-50% d. Th.).

**Methode 2 :** 100 mg (25 10⁻⁵ mol) (**LA.1.3**) werden unter Rückfluß in 2 ml absoluten THF mit 55 µl dest. (75 10⁻⁵ mol) Thionylchlorid über 1h umgesetzt. Das Reaktionsgemisch wird vollständig eingeengt und kurz im HV behandelt. Es werden 2ml abs. tert-Butanol und 42 µl (25 10⁻⁵ mol) Ethyldiisopropylamin zugesetzt und 2h refluxiert. Das Reaktionsgemisch wird in eine gesättigte wässrige NaCl-Lösung gegossen und die wässrige Phase 2x mit 100ml Ethylacetat extrahiert. Die organische Phase wird über MgSO₄ getrocknet und eingeengt. (**LA.1.4**) wird entweder RP-C₁₈-HPLC chromatograpisch zur Homogenität aufgereinigt oder als Rohprodukt (> 95% Gehalt (**LA.1.4**)) in die weitere Reaktion eingesetzt.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.95 (d, 1H, NH), 5.47 (d, 1H, NH-CHOH), 5.23 ('d', 2H COOCH₂), 4.4 ('m' (dt), 2H, CH-CH₂), ³J_{H.H}= 6.67 Hz), 4.23 (t, 1H, CH-CH₂, ³J_{H.H}=6.67 Hz), 1.25 (s, 9H, C(CH₃)₃).- ¹³C-NMR (75 MHz, CDCl₃) : δ = 168.5 (s, COOH), 154.2 (s, NH-COO), 143.7 (s, Fluorenyl-C₆), 141.4 (s, Fluorenyl-C₅), 135.2 (s, Phenyl-H), 128.57/128.4/128.1/127.1/125.1/120.0 (d, Fluorenyl-C / Phenyl-C), 78.4 (d, NH-CHOH), 74.6 (s, C(CH₃)₃), 67.2 (t, COO-CH₂), 67.1 (t CH-CH₂), 47.1 (d, CH-CH₂), 28.2 (q , C(CH₃)₃).- MS (FAB, 3-NBA) : m/z = 461 (27, [M+H]^{⊕}).

### N^{α}-9-Fmoc-α-rac-tert-butoxy-glycin (LA.1.5)^{(AB)}

### Summenformel (C₂₁H₂₃NO₅)

115 mg (25 10⁻⁵ mol) (**LA.1.4**) werden in 3ml abs. Ethanol/Ethylacetat (1:2) gelöst. Es wird eine Spatelspitze Palladium/Aktivkohle (Fluka) zugegeben und 25 min Wasserstoff durch die Lösung geleitet. Der Katalysator wird abfiltriert und (**LA.1.5**) RP-C₁₈-HPLC chromatograpisch isoliert.- Ausbeute : 60.45 mg (70 % d. Th.).- ¹H-NMR (400 MHz, CDCl₃) : δ = 7.81 d, 2H, Fluorenyl-H¹, ³J_{H.H}= 7.30 Hz), 7.67 (d, 2H, Fluorenyl-H⁴, ³J_{H.H}=7.26 Hz), 7.42 (t, 2H, Fluorenyl-H², ³J_{H.H}= 7.30 Hz), 7.25 (t (Feinaufspaltung d), 2H, Fluorenyl-H³, ³J_{H.H}= 7.30), 5.87 (d, 1H, NH), 5.47 (d, 1H, NH-CHOH), 4.4 ('m', 2H, CH-CH₂, ³J_{H.H}= 6.67 Hz), 4.23 (t, 1H, CH-CH₂, ³J_{H.H}=6.67 Hz), 1.25 (s, 9H, C(CH₃)₃).- ¹³C-NMR (75 MHz, CDCl₃) : δ = 168.5 (s, COOH), 154.2 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.4 (d, NH-CHOH), 74.6 (s, C(CH₃)₃), 67.1 (t CH-CH₂), 47.1 (d, CH-CH₂), 28.2 (q , C(CH₃)₃).- MS (FAB, 3-NBA) : m/z = 370 (37, [M+H]^{⊕}).

### N^{ε}-Boc-Lys-Phe-Phe-α-rac-tert-butoxy-glycyl-βAla-OH (LA.1.1)^{(MV)}

### Summenformel (C₃₈H₅₆N₆O₉)

Das geschützte Peptid (**LA.1.1**) wird nach üblichen Peptidsynthesebedingungen an einem o-Chlorotrityl-funktionalisierten Harz unter Verwendung von (**LA.1.5**) aufgebaut und wie üblich vom Träger abgespalten. Die Freisetzung der Aminofunktion des geschützten N.O-Acetals erfolgt hier mit 10% Morpholin/5% Triethylammoniumchlorid/DMF.- MS (FAB, Thioglycerin) : m/z = 740 (5, [M+H]^{⊕}).

### Anwendung

Das geschützte Peptid (**LA.1.1**) zeigt vollständige Stabilität gegenüber 20% Piperidin/DMF (gezeigt durch quantitative UV/VIS-Analytik der einzelnen Kopplungsschritte und durch Behandlung des geschützten Peptides (**LA.1.1**) in Lösung mit dem oben genannten Reagenz). Nach Abspaltung der Hydroxylschutzgruppe nach üblichen Verfahren (und simultan der Boc-Schutzgruppe des Lysyl-Restes) wird das so entschützte Peptid mit Puffersystem (a), (b) und (g) behandelt. In gewünschter Weise zerfällt die entschützte Modellverbindung in das Peptidamid H-Lys-Phe-Phe-NH₂.

### Syntheseweg A / Beispiel 2

### H-Lys(Boc)-Phe-Phe-α-rac-(MOM)oxy-β-trifluoralanin-βAla-OH (LA.2.1)

### Struktur

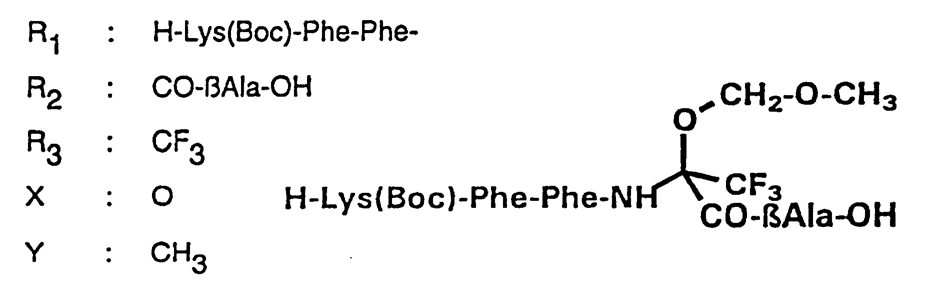

### Syntheseweg

### N^{α}-9-Fmoc-α-hydroxy-β.β.β-trifluoralaninmethylester (LA.2.2)

### Summenformel (C₁₉H₁₆F₃NO₅)

Analog zu (**LA.1.2**) wurde mit Carbamidsäure-9-fluorenylmethylester in Ethylacetat über 4d mit 3.3.3-Trifluorbrenztraubensäuremethylester zur Reaktion gebracht. Das Reaktionsgemisch wird in ein Gemisch aus Diethylether/Petroleumbenzin gegossen und bei -20°C stehengelassen. Das Kristallisat wird abfiltriert und der Überstand eingeengt.- Ausbeute : (75% d. Theorie).- ¹H-NMR (400 MHz, CDCl₃) : δ = 7.81 (d, 2H, Fluorenyl-H¹, ³J_{H.H}= 7.30 Hz), 7.67 (d, 2H, Fluoreny)-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (s, 1H, NH), 4.4 (d, 2H, CH-CH₂), ³J_{H.H}= 6.67 Hz), 4.23 (t, 1H, CH-CH₂, ³J_{H.H}=6.67 Hz), 3.86 (s, 3H, CH₃).- ¹³C-NMR (75 MHz, CDCl₃) : δ = 164.3 (s, COOCH₃), 154.0 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 93.1 (d, NH-C(CF₃)OH), 67.8 (t, CH-CH₂), 54.0 (q, COOCH₃), 47.0 (d, CH-CH₂).- ¹⁹F-NMR (376 MHz, CDCl₃) : δ = -79.4 (s, CF₃).- MS (FAB, 3-NBA) : m/z = 395 (23, [M+H]^{⊕}).

### N^{α}-9-Fmoc-α-(methoxymethyl)oxy-β.β.β-trifluoralaninmethylester (LA.2.3)

### Summenformel (C₂₁H₂₃NO₄S)

(**PA.2.2**) wird in einen Gemisch aus der 10 fachen Menge Fomaldehyddimethylacetal und der gleichen Menge absoluten Chloroform in Gegenwart eines großen Überschusses an Phosphorpentoxid dargestellt. Das Reaktionsgemisch wird in eine gesättigte Natriumchlorid-Lösung gegossen und 2x mit Ethylacetat extrahiert. Die organische Phase wird über Na₂SO₄ getrocknet und eingeengt. Der Rückstand wird in Ethanol gelöst und rasch Wasser zugesetzt. Die milchige Lösung wird vorsichtig zur Hälfte eingeengt und bei 4°C über 4h belassen. Der weiße Feststoff wird abfiltriert und im HV getrocknet.- Ausbeute : (74% d. Theorie).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (s, 1H, NH), 5.05 (d, 1H, O-CH₂-O, ²J_{H.H}= 7.30Hz), 4.82 (d, 1H, O-CH₂-O, ²J_{H.H}= 7.30Hz), 4.4 ('ddd', 2H, CH-CH₂, ³J_{H.H}= 6.70Hz), 4.20 (t, 1H, CH-CH₂, ³J_{H.H}=6.70Hz), 3.86 (s, 3H, CH₃), 3.40 (s, 3H, CH₃O).- ¹³C-NMR (75 MHz, CDCl₃) : δ = 164.3 (s, COOCH₃), 154.0 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 94.2 (d, NH-C(CF₃)OH), 77.5 (t, O-CH₂-O), 67.8 (t, CH-CH₂), 56.6 (q, CH₃O), 54.0 (q, COOCH₃), 47.0 (d, CH-CH₂).- ¹⁹F-NMR (376 MHz, CDCl₃) : δ = -80.1 (s, CF₃).- MS (FAB, 3-NBA) : m/z = 395 (23, [M+H]^{⊕}).

### N^{α}-9-Fmoc-α-(methoxymethyl)oxy-β.β.β-trifluoralanin (LA.2.4)^{(AB)}

### Summenformel (C₂₀H₁₈F₃NO₆)

Die Carboxylfunktion von (**LA.2.3**) wurde in Aceton/Wasser unter Katalyse von LiOH freigesetzt. Das Produkt wurde RP-C₁₈-HPLC chromatograpisch isoliert.-Ausbeute : (65% d. Theorie).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (s, 1H, NH), 5.05 (d, 1H, O-CH₂-O, ²J_{H.H}= 7.30Hz), 4.82 (d, 1H, O-CH₂-O, ²J_{H.H}= 7.30Hz), 4.4 ('ddd', 2H, CH-CH₂, ³J_{H.H}= 6.70Hz), 4.20 (t, 1H, CH-CH₂, ³J_{H.H}=6.70Hz), 3.40 (s, 3H, CH₃O).- ¹³C-NMR (75 MHz, CDCl₃) : δ = 164.3 (s, COOH), 154.1 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 94.2 (d, NH-C(CF₃)OH), 77.0 (t, O-CH₂-O), 67.8 (t, CH-CH₂), 56.6 (q, CH₃O), 54.0 (q, COOCH₃), 47.0 (d, CH-CH₂).- ¹⁹F-NMR (376 MHz, CDCl₃) : δ = -79.4 (s, CF₃).- MS (FAB, 3-NBA) : m/z = 426 (23, [M+H]^{⊕}).

### H-Lys(Boc)-Phe-Phe-α-rac-methoxy-β.-trifluoralanin-βalanin (LA.2.1)^{(MV)}

### Summenformel (C₃₇H₄₂F₃N₆O₁₀)

Nach allgemeinen Peptidsynthesemethoden wird (**LA.2.1**) an einem o-Chlorotrityl-funktionalisierten Harz aufgebaut und als geschütztes Peptid nach bekannten Verfahren abgelöst.- MS (FAB) : M/Z (3-NBA) = 789 ([M+H]^{⊕}).

### Anwendung

Behandlung mit 95% TFA/2.5% TIBS/2.5% Wasser führt zur simultanen Entschützung der BOC-Schutzgruppe des Lysylrestes und der Hydroxylfunktion des N.O-Acetals. Dieses entschützte Peptid zerfällt in gewünschter Weise in das Peptidamid durch Behandlung mit Puffersystem (a) bis (g). Die Reaktion verläuft innerhalb 15min bei 50°C.

### Syntheseweg A / Beispiel 3

### H-Lys(Boc)-Phe-Phe-α-rac-(alkoxymethyl)oxyglycyl-βAla (LA.3.1)^{(MV)}

### Struktur

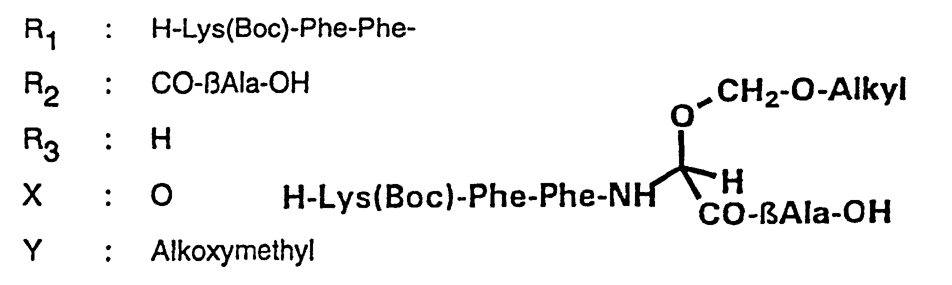

Es wurden verschiedene Schutzgruppen basierend auf einer acetalischen Struktur in die zugrundeliegenden Ankerbaustein eingeführt. Die Stabilität des geschützten N.O-Acetals mit freier Aminofunktion ist so gering, daß das geschützte N.O-Acetal während der basischen Abspaltung der Fmoc-Schutzgruppe im überwiegenden Maße zerfällt, bevor es mit dem folgenden Aminosäurerest zur Reaktion gebracht werden kann. Es können nur Spuren der gewünschten Modellverbindung (**LA.3.1**) isoliert werden. Die Tests erfolgten sowohl in Lösung mit den entsprechenden Benzylester als auch am festen Träger mit Hilfe der Ankerbausteine. Zur Vollständigkeit sollen diese Verbindungen und die entsprechenden Ankerbausteine aufgeführt werden.

### Syntheseweg

### N^{α}-9-Fmoc-α-(methoxymethyl)oxy-glycinbenzylester (LA.3.2)

### Summenformel (C₂₁H₂₃NO₄S)

Analog zu (**LA.2.3**) wird (**LA.3.2**) aus (**LA.1.3**) synthetisiert.- Ausbeute : 1.85 g (92 % d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.95 (d, 1H, NH, ³J_{H.H}=7.31 Hz), 5.47 (d, 1H, NH-CHOH, ³J_{H.H}=7.31 Hz), 5.23 (s, 2H COOCH₂), 4.95 (d, 1H, O-CH₂-O, ²J_{H.H}= 7.26Hz), 4.82 (d, 1H, O-CH₂-O, ²J_{H.H}= 7.24Hz), 4.4 ('m' (dt), 2H, CH-CH₂), ³J_{H.H}= 6.67 Hz), 4.23 (t, 1H, CH-CH₂, ³J_{H.H}=6.67 Hz), 3.40 (s, 3H, CH₃O).- ¹³C-NMR (75 MHz, CDCl₃) : δ = 168.5 (s, COOH), 154.2 (s, NH-COO), 143.7 (s, Fluorenyl-C₆), 141.4 (s, Fluorenyl-C₅), 135.2 (s, Phenyl-H), 128.57/128.4/128.1/127.1/125.1/120.0 (d, Fluorenyl-C / Phenyl-C), 78.4 (d, NH-CHOR), 77.0 (t, O-CH₂-O), 67.2 (t, COO-CH₂), 67.1 (t CH-CH₂), 57.2 (q, CH₃O), 47.1 (d, CH-CH₂).- MS (FAB, 3-NBA) : m/z = 405 (28, [M+H]^{⊕}).

### N^{α}-9-Fmoc-α-(methoxymethyl)oxy-glycin (LA.3.3)^{(AB)}

### Summenformel (C₂₁H₂₃NO₄S)

Analog zu (**LA.1.5**) wird (**LA.3.3**) aus (**LA.3.2**) synthetisiert.- Ausbeute : 1.85 g (92% d. Th.).- ¹H-NMR (400 MHz, CDCl₃) : δ = 7.81 d, 2H, Fluorenyl-H¹, ³J_{H.H}= 7.30 Hz), 7.67 (d, 2H, Fluorenyl-H⁴, ³J_{H.H}=7.26 Hz), 7.42 (t, 2H, Fluorenyl-H², ³J_{H.H}= 7.30 Hz), 7.25 (t (Feinaufspaltung d), 2H, Fluorenyl-H³, ³J_{H.H}= 7.30), 5.87 (d, 1H, NH), 5.47 (d, 1H, NH-CHOH), 4.94 (d, 1H, O-CH₂-O, ²J_{H.H}= 7.20Hz), 4.75 (d, 1H, O-CH₂-O, ²J_{H.H}= 7.20Hz), 4.4 ('m', 2H, CH-CH₂, ³J_{H.H}= 6.67 Hz), 4.23 (t, 1H, CH-CH₂, ³J_{H.H}=6.67 Hz), 3.40 (s, 3H, CH₃O).- ¹³C-NMR (100 MHz, CDCl₃) : δ = 168.5 (s, COOH), 154.2 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.4 (d, NH-CHOH), 77.0 (t, O-CH₂-O), 67.1 (t CH-CH₂), 54.1 (q, CH₃O), 47.1 (d, CH-CH₂).- MS (FAB, 3-NBA) : m/z = 370 (37, [M+H]^{⊕}).

### N^{α}-9-Fmoc-α-(methoxyethoxymethyl)oxy-glycinbenzylester (LA.3.4)

### Summenformel (C₂₁H₂₃NO₄S)

(**LA.3.4**) wird aus (**LA.1.3**) durch Umsetzung mit Methoxyethoxymethylchlorid (Fluka) unter Katalyse von 1.0 eq. Ethyldiisopropylamin in DCM synthetisiert.-Ausbeute : 1.85 g (92% d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.87 (d, 1H, NH), 5.47 (d, 1H, NH-CHOH), 5.23 ('d', 2H, COOCH₂), 4.94 (d, 1H, O-CH₂-O, ²J_{H.H}= 7.20Hz), 4.75 (d, 1H, O-CH₂-O, ²J_{H.H}= 7.20Hz), 4.4 ('m', 2H, CH-CH₂, ³J_{H.H}= 6.67 Hz), 4.23 (t, 1H, CH-CH₂, ³J_{H.H}=6.67 Hz), 3.85 (AB-t, 4H, CH₂-CH₂), 3.40 (s, 3H, CH₃O).- ¹³C-NMR (100 MHz, CDCl₃) : δ = 166.7 (s, COOH), 155.4 (s, NH-COO), 143.6 (s, Fluorenyl-C₆), 141.4 (s, Fluorenyl-C₅), 134.7 (s, Phenyl-H), 128.5-120.0 (5 Signale) (d, Fluorenyl-H/Phenyl-H), 78.9 (d, NH-CHOH), 77.0 (t, O-CH₂-O), 67.9 (t, CH-CH₂), 67.5 (t, COO-CH₂), 67.4 (t, CH₂-CH₂), 46.9 (d, CH-CH₂), 30.9 (q, CH₃O).- MS (FAB, 3-NBA) : m/z = 370 (37, [M+H]^{⊕}).

### N^{α}-9-Fmoc-α-(trimethylsilylethoxymethyl)oxy-glycinbenzylester (LA.3.5)

### Summenformel (C₂₁H₂₃NO₄S)

(**LA.3.4**) wird aus (**LA.1.3**) durch Umsetzung mit Trimethylsilylethoxymethylchlorid (Fluka) unter Katalyse von 1.0 eq. Ethyldiisopropylamin in DCM/DMF = 6/1 synthetisiert.- Ausbeute : 1.85 g (92% d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.87 (d, 1H, NH), 5.47 (d, 1H, NH-CHOH), 5.24 ('d', 2H, COOCH₂), 4.94 (d, 1H, O-CH₂-O, ²J_{H.H}= 7.20Hz), 4.75 (d, 1H, O-CH₂-O, ²J_{H.H}= 7.20Hz), 4.4 ('m', 2H, CH-CH₂, ³J_{H.H}= 6.67 Hz), 3.82 (AB-t, 4H, CH₂-CH₂), 4.23 (t, 1H, CH-CH₂, ³J_{H.H}=6.67 Hz), 0.1 (s, 3H, Si(CH₃)₃).- ¹³C-NMR (100 MHz, CDCl₃) : δ = 166.7 (s, COOH), 155.4 (s, NH-COO), 143.6 (s, Fluorenyl-C₆), 141.4 (s, Fluorenyl-C₅), 134.7 (s, Phenyl-H), 128.5-120.0 (5 Signale) (d, Fluorenyl-H/Phenyl-H), 78.9 (d, NH-CHOH), 77.0 (t, O-CH₂-O), 67.9 (t, CH-CH₂), 67.4 (t, CH₂-CH₂), 67.2 (t, COO-CH₂), 46.9 (d, CH-CH₂), 2.0 (q, Si(CH₃)₃).- MS (FAB, 3-NBA) : m/z = 370 (37, [M+H]^{⊕}).

### N^{α}-9-Fmoc-α-(trimethylsilylethoxymethyl)oxy-glycin (LA.3.6)^{(AB)}

### Summenformel (C₂₁H₂₃NO₄S)

Analog zu (**LA.2.3**) wird (**LA.3.2**) aus (**LA.1.3**) synthetisiert.- Ausbeute : 1.85 g (96% d. Th.).- ¹H-NMR (400 MHz, CDCl₃) : δ = 7.81 d, 2H, Fluorenyl-H¹, ³J_{H.H}= 7.30 Hz), 7.67 (d, 2H, Fluorenyl-H⁴, ³J_{H.H}=7.26 Hz), 7.42 (t, 2H, Fluorenyl-H², ³J_{H.H}= 7.30 Hz), 7.25 (t (Feinaufspaltung d), 2H, Fluorenyl-H³, ³J_{H.H}= 7.30), 5.87 (d, 1H, NH), 5.47 (d, 1H, NH-CHOH), 4.94 (d, 1H, O-CH₂-0, ²J_{H.H}= 7.20Hz), 4.75 (d, 1H, O-CH₂-O, ²J_{H.H}= 7.20Hz), 4.4 ('m', 2H, CH-CH₂, ³J_{H.H}= 6.67 Hz), 3.82 (AB-t, 4H, CH₂-CH₂), 4.23 (t, 1H, CH-CH₂, ³J_{H.H}=6.67 Hz), 0.1 (s, 3H, Si(CH₃)₃).- ¹³C-NMR (100 MHz, CDCl₃) : δ = 168.5 (s, COOH), 154.2 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.9 (d, NH-CHOH), 77.0 (t, O-CH₂-O), 67.9 (t, CH-CH₂), 67.4 (t, CH₂-CH₂), 46.9 (d, CH-CH₂), 2.0 (q, Si(CH₃)₃).

### Syntheseweg A / Beispiel 4

### H-Lys(Boc)-Phe-Phe-α-rac-tert-butyl-dimethylsilyloxyglycyl-βAla (LA.4.1)

### Struktur

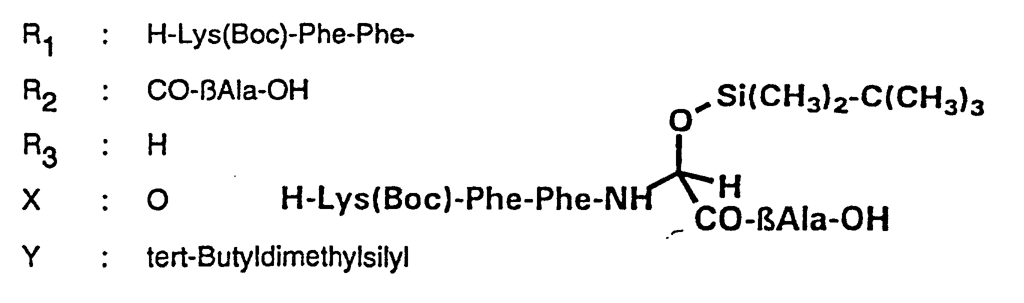

### N^{α}-9-Fmoc-α-tert-butyl-dimethylsilyloxy-glycinbenzylester (LA.4.2)

### Summenformel (C₃₀H₃₅NO₅Si)

100 mg (25 10-5 mol) (**LA.1.3**) und 52.5 mg (37.5 10-5 mol) tert-Butyldimethylsilylchlorid werdem in einem Gemisch aus 2 ml absoluten DMF und 2 ml Dichlormethan unter Erwärmen gelöst. Es werden 42.2 µl Ethyldiisopropylamin zugesetzt und 12 h refluxiert. Das Produkt wird nach üblicher Aufarbeitung RP-C₁₈-HPLC chromatograpisch isoliert.- Ausbeute : 84 mg (63-67% d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.95 (d, 1H, NH), 5.47 (d, 1H, NH-CHOH), 5.23 ('d', 2H COOCH₂), 4.4 ('m' (dt), 2H, CH-CH₂), ³J_{H.H}= 6.67 Hz), 4.23 (t, 1H, CH-CH₂, ³J_{H.H}=6.67 Hz), 0.85 (s, 9H, SiC(CH₃)₃), 0.15 ('d', 6H, Si(CH₃)₂).- ¹³C-NMR (75 MHz, CDCl₃) : δ = 168.5 (s, COOH), 154.2 (s, NH-COO), 143.7 (s, Fluorenyl-C₆), 141.4 (s, Fluorenyl-C₅), 135.2 (s, Phenyl-H), 128.57/128.4/128.1/127.1/125.1/120.0 (d, Fluorenyl-C / Phenyl-C), 78.4 (d, NH-CHOH), 70.1 (s, SiC(CH₃)₃), 67.2 (t, COO-CH₂), 67.1 (t CH-CH₂), 47.1 (d, CH-CH₂), 25.2 (q , C(CH₃)₃), 4.0 (q, Si(CH₃)₂).- MS (FAB, 3-NBA) : m/z = 461 (27, [M+H]^{⊕}).

### N^{α}-9-Fmoc-α-tert-butyl-dimethylsilyloxy-glycin (LA.4.3)^{(AB)}

### Summenformel (C₂₃H₂₉NO₅Si)

Analog zu (**LA.1.5**) wird (**LA.4.2**) in Gegenwart von Pd/Aktivkohle in Ethylacetat/EtOH im Wasserstoffstrom behandelt.- Ausbeute : 570 mg (92% d. Th.; Dichlormethan/Petroleumbenzin ).- ¹H-NMR (400 MHz, CDCl₃) : δ = 7.81 (d, 2H, Fluorenyl-H1, ³J_{H.H}= 7.30 Hz), 7.67 (d, 2H, Fluorenyl-H⁴, ³J_{H.H}=7.26 Hz), 7.4-7.2 (m, 9H, Fluorenyl-H^{2.3}/Phenyl-H), 5.95 (d, 1H, NH), 5.47 (d, 1H, NH-CHOH), 5.23 ('d', 2H COOCH₂), 4.4 ('m' (dt), 2H, CH-CH₂), ³J_{H.H}= 6.67 Hz), 4.23 (t, 1H, CH-CH₂, ³J_{H.H}=6.67 Hz), 0.85 (s, 9H, SiC(CH₃)₃), 0.15 ('d', 6H, Si(CH₃)₂).- ¹³C-NMR (75 MHz, CDCl₃) : δ = 168.5 (s, COOH), 154.2 (s, NH-COO), 143.7 (s, Fluorenyl-C₆), 141.4 (s, Fluorenyl-C₅), 135.2 (s, Phenyl-H), 128.57/128.4/128.1/127.1/125.1/120.0 (d, Fluorenyl-C / Phenyl-C), 78.4 (d, NH-CHOH), 69.9 (s, SiC(CH₃)₃), 67.2 (t, COO-CH₂), 67.1 (t CH-CH₂), 47.1 (d, CH-CH₂), 28.2 (q , C(CH₃)₃), 4.0 (q, Si(CH₃)₂).- MS (FAB, 3-NBA) : m/z = 461 (27, [M+H]^{⊕}).

### H-Lys(Boc)-Phe-Phe-α-rac-(TBDMS)oxyglycyl-βAla-OH (LA.4.1)^{(MV)}

### Summenformel (C₄₀H₅₂N₆O₉Si)

Das geschützte Peptid (**LA.4.1**) wird nach üblichen Peptidsynthesebedingungen an einem o-Chlorotrityl-funktionalisierten Harz unter Verwendung von (LA.4.3) aufgebaut und wie üblich vom Träger abgespalten. Die Freisetzung der Aminofunktion des geschützten N.O-Acetals erfolgt hier mit 10% Morpholin/5% Triethylammoniumchlorid/DMF.- MS (FAB, 3-NBA) : m/z = 788 (27, [M+H]^{⊕}).

### Anwendung

Das geschützte Peptid (**LA.4.1**) zeigt vollständige Stabilität gegenüber 20% Piperidin/DMF (gezeigt durch durch quantitative UV/VIS-Analytik der einzelnen Kopplungsschritte und durch Behandlung des geschützten Peptides (**LA.4.1**) in Lösung mit 20% Piperidin/DMF). Nach Abspaltung der Hydroxylschutzgruppe nach üblichen Verfahren (und simultan der Boc-Schutzgruppe des Lysyl-Restes) wird das entschützte Peptid mit Puffersystem (a), (b) und (g) behandelt. In gewünschter Weise zerfällt die entschützte Modellverbindung in das Peptidamid H-Lys-Phe-Phe-NH₂.

### Syntheseweg A / Beispiel 5

### H-Lys(Boc)-Phe-Phe-α-rac-ethylthio-glycyl-βAla-OH (LA.5.1)

### Struktur

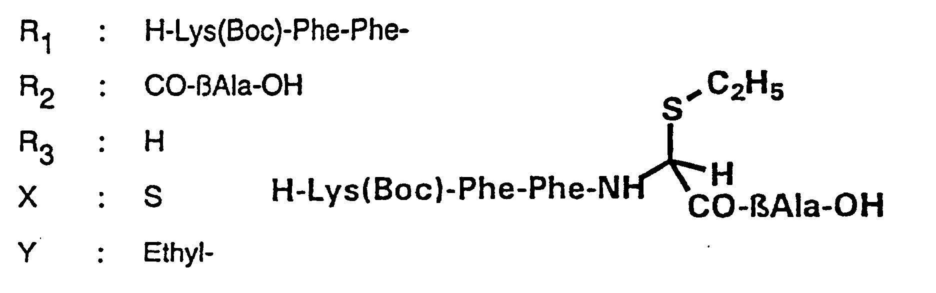

### Syntheseweg

### N^{α}-9-Fmoc-α-ethylthio-glycin (LA.5.2)^{(AB)}

### Summenformel (C₂₀H₂₁NO₄S)

522 mg (1.67 10⁻³ mol) (**LA.1.2**) werden in 1.66 ml Eisessig suspendiert und nacheinander 619 µl (6.67 10⁻³ mol) Ethylmercaptan und 166 µl konz. Schwefelsäure bei 0°C zugegeben. Es wird 1 h bei 0°C und 24h bei RT gerührt, das Reaktionsgemisch in Eiswasser gegossen und 3x mit 100ml Ethylacetat extrahiert. Die organische Phase wird mit gesättigter NaCl-Lösung neutral gewaschen, über Na₂SO₄ getrocknet und eingeengt. Der ölige Rückstand wird in wenig DCM gelöst und durch Zugabe von Petroleumbenzin als weißer Feststoff durch längeres Stehen bei -20°C kristallisiert.- Ausbeute : 570 mg (87% d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (d, 1H, NH), 5.47 (d, 1H, NH-CHOH), 4.40 (d, 2H, CH-CH₂, ³J_{H.H}= 6.67 Hz), 4.23 (t, 1H, CH-CH₂, ³J_{H.H}=6.67 Hz), 2.55 (q, 2H, S-CH₂), 1.23 (t, 3H, CH₃).- ¹³C-NMR (75 MHz, CDCl₃) : δ = 168.9 (s, COOH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.9 (d, NH-CHOH), 68.1 (t, CH-CH₂), 46.8 (d, CH-CH₂), 27.4 (t, S-CH₂), 15.2 (q, CH₃).- MS (FAB, 3-NBA) : m/z = 315 (23, [M+H]^{⊕}).

### H-Lys(Boc)-Phe-Phe-α-rac-ethylthio-glycyl-βAla-OH (LA.5.1)^{(MV)}

### Summenformel (C₃₆H₄₂N₆O₈S)

Das geschützte Modellpeptid (**LA.5.1**) wurde am o-Chlorotrityl-funktionalisierten Polystyrol schrittweise unter Verwendung des Linkerbausteins (**LA.5.2**) synthetisiert. Die Entschützung der Aminofunktion am Linkerbaustein (**LA.5.2**) erfolgt mit 20% Piperidin/DMF. Die Aufreinigung erfolgt RP-C₁₈-HPLC chromatograpisch. Beide Diastereomere sind chromatographisch trennbar.- MS (FAB) : m/z (3-NBA) = 718 ([M+H]^{⊕})

### Anwendung

Bei der Synthese des geschützten Peptides (**LA.5.1**) wurde das am Stickstoff entschützte und das an der Thiolfunktion geschützte N.S-Acetal durchlaufen. Dieses ist gegenüber 20% Piperidin/DMF stabil und läßt sich mit dem folgendem Aminosäurederivat ohne nennenswerte Zerstörung der N.S-Acetal-Struktur zur Reaktion bringen. Das vom Harz abgespaltene Peptid (**LA.5.1**) wurde zudem noch mit dem Abspaltungsreagens 24h behandelt. Es ist keine Veränderung des Eduktes zu beobachten. Die Behandelung von (**LA.5.1**) mit 95% TFA/2.5% TIBS/2.5% H₂O führt zur Abspaltung der Boc-Schutzgruppe im Lysin-Rest. Unter diesen Bedingungen bleibt die Thiolfunktion des N.S-Acetals geschützt. Das partiell entschützte Peptid wird mit einem Überschuß an 2 %igen wässrigen Hg-II-chlorid zusammen mit 10%igem wässriger Essig-säure behandelt. Diese Bedingungen führen zur Überführung des N.S-Acetals in ein N.O-Acetal. Dieses ist stabil unter sauren wässrigen Bedingungen. Das so dargestellte N.O-Acetal zerfällt in gewünschter Weise in das Peptidamid H-Lys-Phe-Phe-NH₂ unter neutralen wässrigen Bedingungen innerhalb von Minuten bei 50°C und ca. 15 min bei RT (Puffersystem : a,b,f,g). Damit eignet sich (**LA.5.2**) als Linkerbaustein in der vorgeschlagenen Weise.

### Syntheseweg A / Beispiel 6

### H-Lys(Boc)-Phe-Phe-α-rac-iso-propylthio-glycyl-βAla-OH (LA.6.1)

### Struktur

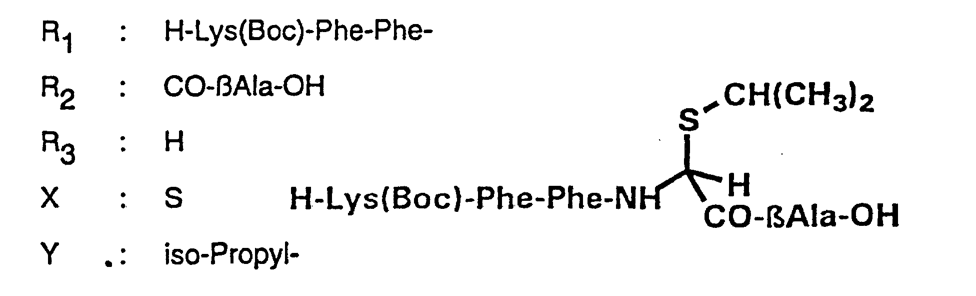

### Syntheseweg

### N^{α}-9-Fmoc-α-isopropylthio-glycin (LA.6.2)^{(AB)}

### Summenformel (C₂₀H₂₁NO₄S)

(**LA.6.2**) wurde analog zu (**LA.5.2**) ausgehend von (**LA.1.2**) durch Umsetzung mit Isopropylmercaptan erhalten. Ausbeute : 613mg (93% d. Th.; Dichlormethan/Petroleumbenzin).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (d, 1H, NH), 5.47 (d, 1H, NH-CHOH), 4.40 (d, 2H, CH-CH₂, ³J_{H.H}= 6.67 Hz), 4.23 (t, 1H, CH-CH₂, ³J_{H.H}=6.67 Hz), 3.11 (heptett, 1H, S-CH), 1.24 (d, 6H, S-CH(CH₃)₂).- ¹³C-NMR (75 MHz, CDCl₃) : δ = 168.9 (s, COOH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.9 (d, NH-CHOH), 68.1 (t, CH-CH₂), 46.8 (d, CH-CH₂), 34.1 (d, S-CH), 15.2 (q, CH₃).- MS (FAB, 3-NBA) : m/z = 315 (23, [M+H]^{⊕}).

### H-Lys(Boc)-Phe-Phe-α-rac-iso-propylthio-glycyl-βAla-OH (LA.6.1)^{(MV)}

### Summenformel (C₃₇H₄₄N₆O₈S)

Nach allgemeinen Methoden wurde an einem o-Chlorotrityl-derivatisierten Polystyrolharz aufgebaut.- MS (FAB) : M/Z (3-NBA) = 732 (14, [M+H]^{⊕}).

### Anwendung

Die Tests an der Modellverbindung (**LA.6.1**) erfolgen in dergleichen Weise wie an der Modellverbindung (**LA.5.1**) (siehe oben). Die Modellverbindung mit geschützter Thiolfunktion des N.S-Acetals zeigt im Verlauf der Synthese und bei Tests in Lösung vollständige Stabilität gegenüber dem Reagenz 20% Piperidin/DMF. Das nach Behandlung mit Quecksilbersalzen gebildete ungeschützte N.O-Acetal (siehe oben) erfällt in wässriger, neutraler Lösung in das gewünschte Peptidamid.

### Syntheseweg A / Beispiel 7

### H-Lys(Boc)-Phe-Phe-α-rac-tert-butylthio-glycyl-βAla-OH (LA.7.1)

### Struktur

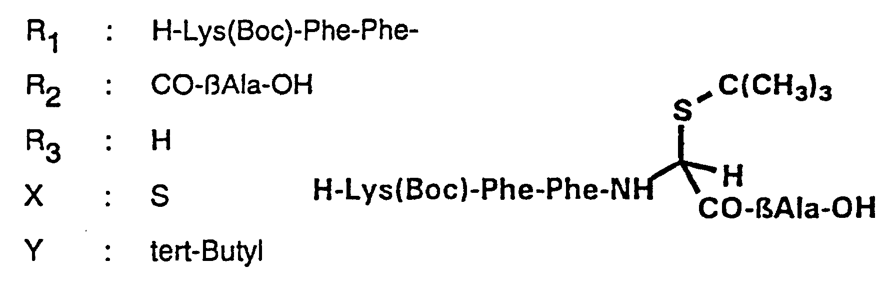

### Syntheseweg

### N^{α}-9-Fmoc-α-rac-tert-butylthio-glycin (LA.7.2)^{(AB)}

### Summenformel (C₂₁H₂₃NO₄S)

(**LA.7.2**) wurde analog zu (**LA.5.2**) ausgehend von (**LA.7.2**) durch Umsetzung mit tert-Butylmercaptan erhalten. Ausbeute : 612mg (92% d. Th.; Dichlormethan/Petroleumbenzin).- ¹H-NMR (400 MHz, CDCl₃) : δ = 7.81 (d, 2H, Fluorenyl-H¹, ³J_{H.H}=7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.95 (d, 1H, NH), 5.47 (d, 1H, NH-CHOH), 4.40 (d, 2H, CH-CH₂, ³J_{H.H}= 6.67 Hz), 4.23 (t, 1H, CH-CH₂, ³J_{H.H}=6.67 Hz), 1.25 (s, 9H, S-C(CH₃)₃).- ¹³C-NMR (75 MHz, CDCl₃) : δ = 168.9 (s, COOH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.9 (d, NH-CHOH), 68.1 (t, CH-CH₂), 46.8 (d, CH-CH₂), 37.3 (d, S-C(CH₃)₃), 15.2 (q, S-C(CH₃)₃).- MS (FAB, 3-NBA) : m/z = 315 (23, [M+H]^{⊕}).

### H-Lys(Boc)-Phe-Phe-α-rac-tert-butylthio-glycyl-βalanin (LA.7.1)^{(MV)}

### Summenformel (C₃₈H₄₆N₆O₈S)

(**LA.7.1**) wurde nach allgemeinen Peptidsynthesemethoden schrittweise unter Verwendung von (**LA.7.2**) an einem o-Chlorotrityl-derivatisierten Polystyrolharz aufgebaut.- Die Aminofunktion des geschützten N.S-Acetals wurde durch Behandlung mit 20% Piperidin/ DMF freigesetzt.- MS (FAB) : M/Z (3-NBA) = 746 ([M+H]^{⊕}).

### Anwendung

Die Tests an der Modellverbindung (**LA.7.1**) erfolgen in der gleichen Weise wie an der Modellverbindung (**LA.5.1**) (siehe oben). Die Modellverbindung mit geschützter Thiolfunktion des N.S-Acetals zeigt im Verlauf der Synthese und bei Tests in Lösung Stabilität gegenüber dem Reagenz 20% Piperidin/DMF. Das nach Behandlung mit Quecksilbersalzen gebildete ungeschützte N.O-Acetal (siehe oben) erfällt in wässriger, neutraler Lösung in das gewünschte Peptidamid. Das zugrundeliegende N.S-Acetal mit freier Thiolfunktion läßt sich mit dem Reagenz 15% Trifluormethansulfonsäure/80% Trifluoressigsäure/2.5% TIBS/2.5% Wasser freisetzen und chromatographisch isolieren (Nachweis über MS-FAB). Das entschützte N.S-Acetal zerfällt durch Behandlung mit Puffersystem (b) und (g) innerhalb 20min in gewünschter Weise in das Peptidamid H-Lys-Phe-Phe-NH₂.

### Ankergruppe für N^{α}-Boc/Bzl-Festphasenpeptidsynthese

### Syntheseweg A / Beispiel 8

### H-Lys(Boc)-Phe-Phe-α-rac-tert-butylthio-glycyl-βAla-TentaGel (LA.8.1)

### Struktur

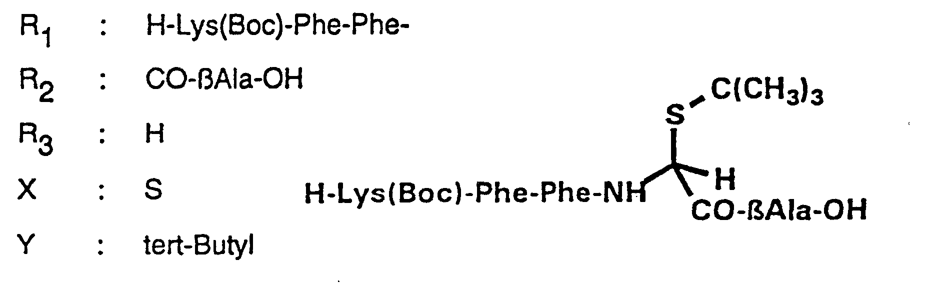

### Syntheseweg

### N^{α}-Boc-α-rac-hydroxy-glycin (LA.8.2)^{(AB)}

### Summenformel (C₂₁H₂₃NO₄S)

(**LA.8.2**) wurde analog zu (**LA.1.2**) ausgehend von Carbamidsäure-tert-butylester durch Umsetzung mit 2.5 Äqu. Glyoxalsäure in Diethylether/THF = 2:3 erhalten. Ausbeute : 87% d. Th.; weißer Feststoff / Petroleumbenzin).- ¹H-NMR (400 MHz, CDCl₃) : δ = 5.27 (s, br, 1H, NH-CH-OH), 1.41 (s, 9H, C(CH₃)₃).- ¹³C-NMR (100 MHz, CDCl₃) : δ = 175.5 (s, COOH), 154.7 (s, NH-COO), 81.1 (s, C(CH₃)₃), 54.1 (d, NH-CH-OH), 32.2 (q, C(CH₃)₃).

### N^{α}-Boc-α-rac-tert-butylthio-glycin (LA.8.3)^{(AB)}

### Summenformel (C₂₁H₂₃NO₄S)

(**LA.8.3**) wurde analog zu (**LA.7.2**) ausgehend von (**LA.8.2**) durch Umsetzung mit 4 Äqu. tert-Butylmercaptan in Eisessig (RZ = 3d) erhalten. Ausbeute : 56% d. Th.; weißer Feststoff, Dichlormethan/Petroleumbenzin, -20°C (14d)).- Smp.: 101°C; ¹H-NMR (400 MHz, CDCl₃) : δ = 5.27 (s, br, 1H, NH-CH-OH), 1.47 (s, 9H, SC(CH₃)₃), 1.41 (s, 9H, NHCOOC(CH₃)₃).- ¹³C-NMR (100 MHz, CDCl₃): δ = 175.4 (s, COOH), 154.3 (s, NH-COO), 81.5 (s, C(CH₃)₃), 54.1 (d, NH-CH-OH), 46.3 (s, SC(CH₃)₃), 32.2 (q, C(CH₃)₃), 28.1 (q, C(CH₃)₃).

### H-Lys(Boc)-Phe-Phe-α-rac-tert-butylthio-glycyl-βAla-TentaGel (LA.8.1)^{(MV)}

### Summenformel (C₃₈H₄₆N₆O₈S)

(**LA.8.1**) wurde nach den allgemeinen Peptidsynthesemethoden der Boc SPPS und Fmoc SPPS schrittweise unter Verwendung von (**LA.8.3**) an einem Ethylenglycol-styrol-Pfropfpolymeren (TentaGel S Amine) aufgebaut.- Dazu wurde der Festphasenträger mit Fmoc-βAla-OH unter Aktivierung mit DIC/HOBt nach den Methoden der Fmoc SPPS funktionalisiert und die Aminofunktion mit 20% Piperidin freigesetzt (Beladung 0.24 mmol/g). (**LA.8.3**) wurde unter Aktivierung von DIC/HOBt in DMF gekoppelt, der Festphasenträger sukzessive mit DMF und DCM gewaschen und die Aminofunktion mit 55% TFA/DCM (2.5% TIBS, 2.5% Wasser) (20 min) entschützt. Der Festphasenträger wird erneut mit DCM gewaschen und Boc-Phe-OH unter Aktivierung von DIC/HOBt in DCM unter Zugabe von 1Äqu. DIEA gekoppelt (30min). Die Reagenzien werden durch Waschen mit DCM entfernt und die Aminofunktion mit 55% TFA/DCM (2.5% TIBS, 2.5% Wasser) entschützt. Es erfolgt die erneute Kopplung von Boc-Phe-OH und Freisetzung der Aminofunktion. Es wird Fmoc-Lys(Boc)-OH unter Aktivierung von DIC/HOBt in DMF gekoppelt und die Beladung des Trägermaterials durch quantitative Abspaltung von Fmoc bestimmt (0.23 mmol/g). Das Trägermaterial wird mit DCM gewaschen und die Peptidsequenz mit 95% TFA/5% TFMSA (2.5% TIBS, 2.5% Wasser) entschützt. Nach je 3maligen Waschen mit MeOH/Wasser = 1:1 (1 % HCl) und 1 M AcOH wird das Trägermaterial im HV 6h getrocknet und das Peptid H-Lys-Phe-Phe-NH2 bei 37oC vom Festphasenträger mit 10 mM KH₂PO₄/Na₂HPO₄ (pH 7.5) eluiert (Homogen nach RP-C18-HPLC und MALDI-TOF-MS (Matrix Sinapinsäure)).

### Syntheseweg B / Beispiel 1

### N^{α}-Ac-Phe-α-methoxy-glycinmethylester (LB.1.1)^{(MV)}

### Struktur

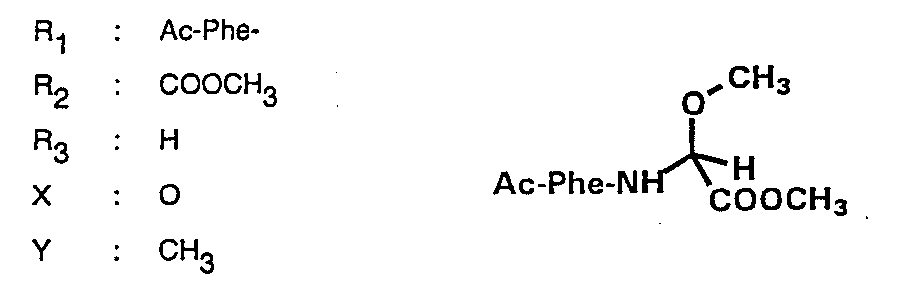

### Syntheseweg

### N^{α}-Acetyl-L-phenylalanyl-α-rac-hydroxy-glycin (LB.1.2)

### Summenformel (C₁₃H₁₆N₂O₅)

103.1 mg (50 10⁻⁵ mol) N^{α}-Acetyl-phenylalanylamid werden zusammen mit 92 mg (100 10⁻⁵ mol) Gyoxalsäure-hydrat in 5 ml absoluten Dioxan bei RT über 2d gerührt. Das Reaktionsgemisch aus den diastereomeren Verbindungen (**LB.1.2**) wird eingeengt und RP-C₁₈-HPLC chromatograpisch getrennt.- Ausbeute : 131 mg (94% d. Th.).- ¹H-NMR (400 MHz, D₂O) : δ = 7.40-7.15 (m, 5H, Phenyl-H), 5.59 (d, 1H, NH-CH-OH), 4.61 (m, 1H, NH-CH-CO), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz), 2.10 (s, 3H, CH₃).- ¹³C-NMR (75 MHz, D₂O) : δ = 174.8 (s, CH₃-CO), 174.2 (s, COOH), 173.16/172.97^{[dia]}(s, CO-NH), 137.2 (s, Phenyl-H), 130.0 (d, Phenyl-H), 129.5 (d, Phenyl-H), 127.9 (d, Phenyl-H), 123.5 (s, Phenyl-H), 72.1 (d, NH-CHOH), 55.8/55.7^{[dia]} (d, NH-CH-CO), 37.8/37.7^{[dia]} (t, CH₂-C₆H₅), 22.4 (q, CH₃).

### N^{α}-Acetyl-L-phenylalanyl-α-rac-methoxy-glycinmethylester (LB.1.1)^{(MV)}

### Summenformel (C₁₅H₂₀N₂O₅)

70.1 mg (25 10⁻⁵ mol) (**LB.1.2**) werden analog zu (**PA.1.3**) in Methanol umgesetzt.- Ausbeute: 65 mg (88% d. Th. / farbloses Öl).- ¹H-NMR (400 MHz, CDCl₃) : δ = 7.40-7.15 (m, 5H, Phenyl-H), 5.59 (d, 1H, NH-CH-OH), 4.61 (m, 1H, NH-CH-CO), 3.81 (s, 3H, CH₃), 3.42 (s, 3H, CH₃), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz), 2.10 (s, 3H, CH₃).- ¹³C-NMR (75 MHz, CDCl₃) : δ = 174.8 (s, CH₃-CO), 174.2 (s, COOH), 173.16/172.97^{[dia]}(s, CO-NH), 137.2 (s, Phenyl-H), 130.05 (d, Phenyl-H), 129.5 (d, Phenyl-H), 127.5 (d, Phenyl-H), 72.1 (d, NH-CHOH), 55.7/55.6^{[dia]} (d, NH-CH-CO), 37.8/37.7^{[dia]} (t, CH₂-C₆H₅), 22.4 (q, CH₃).- MS (FAB) : m/z = 309 (15, [M+H]^{⊕}).

### Anwendung

Die Stabilität von (**LB.1.1**) wurde in 20% Piperidin/DMF (übliche Bedingungen der Synthese an R₁ im Verlauf der N^{α}-Fmoc/tBu-Festphasenpeptidsynthese) bei RT über 2d getestet. (**LB.1.1**) zeigt sich vollständig stabil unter diesen Bedingungen. Nach Freisetzung der Hydroxylfunktion des N.O-Acetals wird das Reaktionsprodukt mit Puffersystem (a) und (b) bei RT (Zerfall nach 20min), 37°C (Zerfall nach 5min) und 50°C (Zerfall nach 5min) behandelt. In allen Fällen zerfällt das N.O-Acetal mit freier Hydroxylfunktion schnell und quantitativ in das erwünschte Ac-Phe-NH₂.

### Syntheseweg B / Beispiel 2

### H-Lys(Boc)-Phe-Phe-α-rac-alkyl/arylthio-glycyl-βAla-OH (LB.2.1)

### Struktur

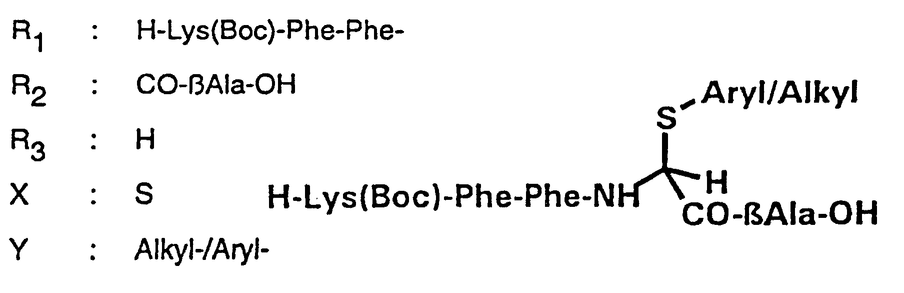

### Syntheseweg

### N^{α}-9-Fmoc-L-phenylalaninamid (LB.2.2)

### Summenformel (C₂₄H₂₂N₂O₃)

Zu einer Lösung von Phenylalanylamid in Dioxan / 10% Na₂CO₃ wird langsam eine Lösung von Chlorameisensäre-9-fluorenylmethylester in Dioxan bei 0°C eingetropft. Die Lösung wird noch 1h bei 0°C, dann weitere 15h bei RT gerührt.Der Feststoff wird abgenutscht, mit Wasser und Petroleumbenzin gewaschen und im Hochvakuum getrocknet.- Ausbeute : (98% d. Th.).- ¹H-NMR (400 MHz, DMSO-d₆) : δ = 7.81 (d, 2H, Fluorenyl-H¹, ³J_{H.H}= 7.30 Hz), 7.67 ('m', 2H, Fluorenyl-H⁴, ³J_{H.H}=7.26 Hz), 7.40-7.15 (m, 9H, Fluorenyl-H /Phenyl-H), 6.1 (d, 1H, NH), 5.5 (s (br), 2H, NH₂), 4.40-4.27 (m, 3H, NH-CH-CO / CH-CH₂), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz).- ¹³C-NMR (75 MHz, DMSO-d₆) : δ = 172.1 (s, CO-NH₂), 156.4 (s, NH-COO), 137.2 (s, Phenyl-H), 130.0-120.5 (5 Signale) (d, Fluorenyl-H / Phenyl-H), 57.1 (d, NH-CH-CO), 37.9 (t, CH₂-C₆H₅).

### N^{α}-9-Fmoc-glycinamid (LB.2.3)

### Summenformel (C₁₆H₁₆N₂O₃)

Analog zu (**LB.2.2**) wird (**LB.2.2**) synthetisiert.- Ausbeute : (97 % d. Theorie).-¹H-NMR (400 MHz, DMSO-d₆) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 7.2 (s (br), 1H, NH₂), 6.9 (t (br), 1H, CO-NH), 4.35 (d, 2H, CH-CH₂), ³J_{H.H}= 6.67 Hz), 4.23 (t, 1H, CH-CH₂, ³J_{H.H}=6.67 Hz), 3.52 (d, 2H, NH-CH₂-CO, ³J_{H.H}= 7.1Hz).-¹³C-NMR (75 MHz, DMSO-d₆) : δ = 168.9 (s, COOH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 78.9 (d, NH-CHOH), 68.1 (t, CH-CH₂), 66.2 (t, NH-CH₂-CO), 46.8 (d, CH-CH₂).

### N^{α}-9-Fmoc-L-phenylalanyl-rac-α-hydroxy-glycin (LB.2.4)

### Summenformel (C₂₆H₂₄N₂O₆)

97 mg (25 10⁻⁵ mol) (**LB.2.2**) werden mit 92 mg (100 10⁻⁵ mol) Glyoxalsäuremonohydrat in 5 ml THF über 24h refluxiert. Das Reaktionsgemisch wird in Ethylacetat gegossen und 3x gegen gesättigte NaCl-Lösung extrahiert. Die organische Phase wird über Na₂SO₄ getrocknet, eingeengt und der Rückstand aus Dichlormethan/ Petroleumbenzin kristallisiert.- Ausbeute : 104 mg (90% d. Thorie - weißer Feststoff).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 9H, Fluorenyl-H / Phenyl-H), 6.1 (d, 1H, NH), 5.47 (d, 1H, NH-CH-OH), 4.40-4.27 (m, 3H, NH-CH-CO / CH-CH₂), 4.23 (t, 1H, CH-CH₂, ³J_{H.H}=6.67 Hz), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz).- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 130.0-120.5 (5 Signale) (d, Fluorenyl-H / Phenyl-H), 72.1 (d, NH-CHOH), 68.1 (t, CH-CH₂), 56.6/55.4^{[dia]} (d, NH-CH-CO), 47.3 (d, CH-CH₂), 37.9 (t, CH₂-C₆H₅).- MS (FAB, Thioglycerin) : m/z = 461 (15, [M+H]^{⊕}).

### N^{α}-9-Fmoc-glycyl-rac-α-hydroxy-glycin (LB.2.5)

### Summenformel (C₁₉H₁₈N₂O₆)

Analog zu (**LB.2.4**) wird (**LB.2.5**) synthetisiert.- Ausbeute 64 mg (68% d. Theorie - weißer Feststoff).- ¹H-NMR (300 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H^{2 3}J_{H.H}= 7.30 Hz), 7.25 (t, 2H, Fluorenyl-H³, ³J_{H.H}= 7.30), 5.90 (t (br), 1H, CO-NH), 4.35 (d, 2H, CH-CH₂), ³J_{H.H}= 6.67 Hz), 4.23 (t, 1H, CH-CH₂, ³J_{H.H}=6.67 Hz), 3.52 (d, 2H, NH-CH₂-CO, ³J_{H.H}= 7.1Hz).- ¹³C-NMR (75 MHz, CDCl₃) : δ = 171.4 (s, COOH), 168.9 (s, CONH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 77.4 (d, NH-CHOH), 68.1 (t, CH-CH₂), 66.2 (t, NH-CH₂-CO), 46.8 (d, CH-CH₂).- MS (FAB, 3-NBA) : m/z = 271 (5, [M+H]^{⊕}).

### N^{α}-9-Fmoc-L-phenylalanyl-rac-α-hydroxy-glycinbenzylester (LB.2.6)

### Summenformel (C₂₁H₂₃NO₄S)

(**LB.2.6**) wird analog zu (**LA.1.3**) durch direkte Umsetzung von (1) mit Benzylbromid und Cäsiumcarbonat in DMF synthetisiert.- Ausbeute : 64% d. Theorie - weißer Feststoff.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H^{4 3}J_{H.H}=7.26 Hz), 7.40-7.15 (m, 14H, Fluorenyl-H / Phenyl-H), 6.1 (d, 1H, NH), 5.47 (d, 1H, NH-CH-OH), 5.23 ('d', 2H COOCH₂), 4.40-4.27 (m, 3H, NH-CH-CO / CH-CH₂), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz).- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 135.2 (s, Phenyl-H), 130.0-120.5 (8 Signale/z.T aufgespalten^{[dia]}) (d, Fluorenyl-H / Phenyl-H), 72.1 (d, NH-CHOH), 68.1 (t, CH-CH₂), 67.5 (t, COO-CH₂), 56.6/55.4^{[dia]} (d, NH-CH-CO), 47.3 (d, CH-CH₂), 37.9 (t, CH₂-C₆H₅).- MS (FAB, Thioglycerin) : m/z = 461 (15, [M+H]^{⊕}).

Die Alkyl-/Arylthioverbindungen werden analog zu (**LA.5.2**) aus (**LB.2.4**) und den entsprechenden Thiolen erhalten. Die Daten werden im Folgenden wiedergegeben. Die Verbindungen verhalten sich analog zu (**LA.5.2-LA.7.2**). Durch Behandlung mit Hg-II-Salzen gehen die entsprechenden Modellverbindungen in die entschützten N.O-Acetale über. (**LB.2.9**) kann durch 95 % TFA/2.5 % TIBS/2.5 % Wasser direkt in das entsprechende entschützte N.S-Acetal (freie Thiolfunktion) überführt werden. Dieses zerfällt in gewünschter Weise in das Peptidamid H-Lys-Phe-Phe-NH₂.

### N^{α}-9-Fmoc-L-phenylalanyl-rac-α-isopropylthio-glycin (LB.2.7)^{(AB)}

### Summenformel (C₂₁H₂₃NO₄S)

Ausbeute : 90% d. Theorie.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 9H, Fluorenyl-H / Phenyl-H), 6.1 (d, 1H, NH), 4.95 (d, 1H, NH-CH-S), 4.40-4.27 (m, 3H, NH-CH-CO / CH-CH₂), 4.20 (t, 1H, CH-CH₂, ³J_{H.H}=6.70Hz), 3.22 (heptett, 1H, S-CH, ³J_{H.H}= 6.74Hz), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz), 1.20 (d, 6H, S-CH(CH₃)₂, ³J_{H.H}= 6.74Hz).-¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 130.0-120.5 (5 Signale) (d, Fluorenyl-H / Phenyl-H), 72.1 (d, NH-CHOH), 68.1 (t, CH-CH₂), 56.6/55.4^{[dia]} (d, NH-CH-CO), 47.3 (d, CH-CH₂), 37.9 (t, CH₂-C₆H₅), 34.1 (d, S-CH), 15.2 (q, CH₃).- MS (FAB, Thioglycerin) : m/z = 461 (15, [M+H]^{⊕}).

### N^{α}-9-Fmoc-L-phenylalanyl-rac-α-benzylthio-glycin (LB.2.8)^{(AB)}

### Summenformel (C₃₃H₃₀N₂O₅S)

Ausbeute : 90% d. Theorie.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.00 (m, 14H, Fluorenyl-H / Phenyl-H), 6.1 (d, 1H, NH), 4.85 (d, 1H, NH-CH-OH), 4.40-4.27 (m, 3H, NH-CH-CO / CH-CH₂), 4.15 (t, 1H, CH-CH₂, ³J_{H.H}=6.70Hz), 3.73[dia] ('d', 2H, S-CH₂-C₆H₅), 3.00 (m, 2H, CH₂-C₆H₅).-¹³C-NMR (75 MHz, CDCl₃) : δ = 174.4 (s, COOH), 170.8/170.6^{[dia]} (s, CO-NH), 156.4 (s, NH-COO), 143.7 (s, Fluorenyl-C₆), 141.4 (s, Fluorenyl-C₅), 137.2 (s, Phenyl-H), 135.9/135.8^{[dia]} (s, Phenyl-H), 130.0-120.5^{[dia]} (14 Signale) (d, Fluorenyl-H / Phenyl-H), 7.6/67.3^{[dia]} (t, CH-CH₂), 56.8/55.5^{[dia]} (d, NH-CH-S) 53.7/53.3^{[dia]} (NH-CH-CO), 47.3 (d, CH-CH₂), 39.0/38.2^{[dia]} (t, CH₂-C₆H₅), 35.4 (t, S-CH₂-C₆H₅).

### N^{α}-9-Fmoc-L-phenylalanyl-rac-α-triphenylmethylthio-glycin (LB.2.9)^{(AB)}

### Summenformel (C₄₅H₃₈N₂O₅S)

Ausbeute : 45 % d. Theorie.- ¹H-NMR (400 MHz, CDCl₃) : δ = 7.81 (d, 2H, Fluorenyl-H¹, ³J_{H.H}= 7.30 Hz), 7.67 ('dd', 2H, Fluorenyl-H⁴, ³J_{H.H}=7.26 Hz), 7.50-7.00 (m, 24H, Fluorenyl-H / Phenyl-H / Trityl-H), 4.95 (d, 1H, NH-CH-S), 4.40-4.27 (m, 3H, NH-CH-CO / CH-CH₂), 4.15 (t, 1H, CH-CH₂, ³J_{H.H}=6.70Hz), 3.10 (m, 2H, CH₂-C₆H₅), .- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 134.7 (s, Trityl-H), 130.0-120.5 (8 Signale) (d, Fluorenyl-C / Phenyl-C / Trityl-C),68.1 (t, CH-CH₂), 56.6/55.4^{[dia]} (d, NH-CH-CO), 53.8/53.5^{[dia]} (d, NH-CH-S) 47.3 (d, CH-CH₂), 37.9 (t, CH₂-C₆H₅), 36.0 (t, S-C(C₆H₅)₃)..- MS (FAB, Thioglycerin) : m/z = 719 (15, [M+H]^{⊕}).

### Syntheseweg B / Beispiel 3

### H-Lys(Boc)-Phe-Phe-α-rac-(methoxymethyl)oxyglycyl-βAla-OH (LB.3.1)

### Struktur

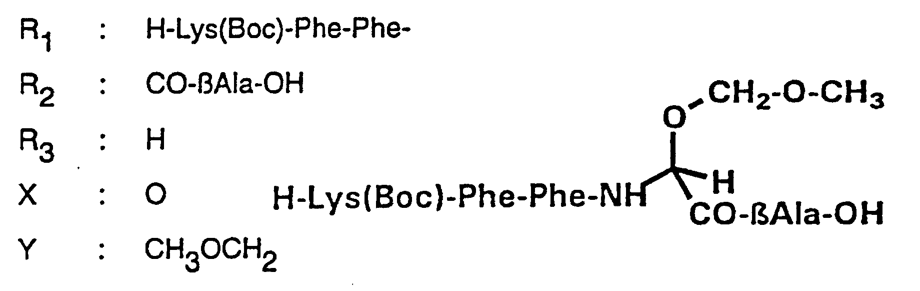

### Syntheseweg

### N^{α}-9-Fmoc-L-phenylalanyl-rac-α-(methoxymethyl)oxy-glycin (LB.3.2)^{(AB)}

### Summenformel (C₂₁H₂₃NO₄S)

(**LB.3.2**) wird analog zu (**LB.3.4**) durch Umsetzung von (**LB.2.4**) mit Formaldehyddimethylacetal synthetisiert.- Ausbeute : 67 % d. Theorie.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 9H, Fluorenyl-H /Phenyl-H), 6.5(d, 1H, NH), 6.1 (d, 1H, NH), 5.47 (d, 1H, NH-CH-O), 5.00 (d, 1H, O-CH₂-O, ²J_{H.H}= 7.30Hz), 4.90 (d, 1H, O-CH₂-O, ²J_{H.H}= 7.30Hz), 4.40-4.27 (m, 3H, NH-CH-CO / CH-CH₂), 4.15 (t, 1H, CH-CH₂, ³J_{H.H}=6.70Hz), 3.45 (s, 3H, CH₃O), 3.10 (m, 1H, CH₂-C₆H₅).- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 130.0-120.5 (5 Signale) (d, Fluorenyl-H / Phenyl-H), 77.0 (t, O-CH₂-O), 72.1 (d, NH-CHO), 68.1 (t, CH-CH₂), 56.6/55.4^{[dia]} (d, NH-CH-CO), 51.6 (q, CH₃O), 47.3 (d, CH-CH₂).- MS (FAB, Thioglycerin) : m/z = 461 (15, [M+H]^{⊕}).

### H-Lys(Boc)-Phe-Phe-α-rac-(methoxymethyl)oxyglycyl-βAla-OH (LB.3.1)^{(MV)}

### Summenformel (C₃₆H₄₂N₆O₁₀)

Nach allgemeinen Peptidsynthesemethoden wird (**LA.3.1**) an einem o-Chlorotrityl-funktionalisierten Harz aufgebaut und als geschütztes Peptid nach bekannten Verfahren abgelöst.- MS (FAB) : M/Z. (3-NBA) = 718 ([M+H]^{⊕}).

### Weitere Beispiele :

Zusätzlich zu (**LB.3.2**) wurden weitere bi- und trifunktionelle Aminosäure in Form ihrer in der Seitenkette geschützten und kommerziell zugänglichen Amide zu den entsprechenden Ankerbausteinen mit MOM geschütztem N.O-Acetal umgesetzt. Die Reaktionssequenz entspricht (**LB.3.2**). Die säurelabilen Seitenkettenfunktionen sind unter den aciden Bedingungen zur Einführung der N.OR-acetalischen Ankergruppierung stabil. Es wurden synthetisiert :
N^{α}-9-Fmoc-L-Ile-rac-α-(methoxymethyl)oxy-glycin (verzweigte bifunktionelle AS)
N^{α}-9-Fmoc-D-Thr(tBu)-rac-α-(methoxymethyl)oxy-glycin (Alkoholfunktion)
N^{α}-9-Fmoc-L-Glu(tBu)-rac-α-(methoxymethyl)oxy-glycin (Carboxylatfunktion)
N^{α}-9-Fmoc-L-Cys(Trt)-rac-α-(methoxymethyl)oxy-glycin (Thiolfunktion)
N^{α}-9-Fmoc-L-Lys(Boc)-rac-α-(methoxymethyl)oxy-glycin (primäres Amin)

Exemplarisch werden hier die experimentellen Daten für N^{α}-9-Fmoc-D-Thr(tBu)-rac-α-(methoxymethyl)oxy-glycin angegeben.

### N^{α}-9-Fmoc-D-Thr(tBu)-NH₂ (LB.3.3)

### Summenformel (C₂₃H₂₈N₂O₄)

Analog zu (**LB.2.2**) wird (**LB.3.3**) synthetisiert.- Ausbeute (68% d. Theorie - weißer Feststoff).- ¹H-NMR (300 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.90 (t (br), 1H, CO-NH), 4.35 (d, 2H, CH-CH₂), ³J_{H.H}= 6.67 Hz), 4.28 (q, 1H, CH₃-CHOH, ³J_{H.H}= 6.67 Hz) 4.23 (t, 1H, CH-CH₂, ³J_{H.H}=6.67 Hz), 3.58 (d, 1H, NH-CH-CO, ³J_{H.H}= 7.1Hz), 1.35 (d, 3H, CH₃-CHOH, ³J_{H.H}= 6.67 Hz), 1.23 (s, 9H, C(CH₃)₃).- ¹³C-NMR (75 MHz, CDCl₃) : δ = 176.2 (s, COOH), 168.9 (s, CONH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 74.6 (s, C(CH₃)₃), 68.1 (t, CH-CH₂), 67.4 (d, CH₃-CHOH), 62.2 (t, NH-CH-CO), 46.8 (d, CH-CH₂), 28.2 (q, C(CH₃)₃), 20.4 (q, CH₃-CHOH).- MS (FAB, 3-NBA) : m/z = 271 (5, [M+H]^{⊕}).

### N^{α}-9-Fmoc-D-Thr(tBu)-rac-α-hydroxy-glycin (LB.3.4)

### Summenformel (C₂₅H₃₀N₂O₇)

Analog zu (**LB.2.3**) wird (**LB.3.4**) synthetisiert.- Ausbeute (98% d. Theorie - weißer Feststoff).- ¹H-NMR (300 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30), 5.90 (t (br), 1H, CO-NH), 4.35 (d, 2H, CH-CH₂), ³J_{H.H}= 6.67 Hz), 4.28 (q, 1H, CH₃-CHOH, ³J_{H.H}= 6.67 Hz) 4.23 (t, 1H, CH-CH₂, ³J_{H.H}=6.67 Hz), 3.58 (d, 2H, NH-CH-CO, ³J_{H.H}= 7.1Hz), 1.35 (d, 3H, CH₃-CHOH, ³J_{H.H}= 6.67 Hz).-¹³C-NMR (75 MHz, CDCl₃) : δ = 176.2 (s, COOH), 168.9 (s, CONH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 77.4 (d, NH-CHOH), 74.6 (s, C(CH₃)₃), 68.1 (t, CH-CH₂), 67.4 (d, CH₃-CHOH), 62.2 (t, NH-CH-CO), 46.8 (d, CH-CH₂), 28.2 (q, C(CH₃)₃), 20.4 (q, CH₃-CHOH).- MS (FAB, 3-NBA) : m/z = 271 (5, [M+H]^{⊕}).

### N^{α}-9-Fmoc-D-Thr(tBu)-rac-α-(methoxymethyl)oxy-glycin (LB.3.5)

### Summenformel (C₂₇H₃₄N₂O₈)

Analog zu (**76**) wird (**79**) synthetisiert.- Ausbeute (86% d. Theorie - weißer Feststoff).- ¹H-NMR (300 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.4 (t, 2H, Fluorenyl-H², ³J_{H.H}= 7.30 Hz), 7.25 (t, 2H, Fluorenyl-H³, ³J_{H.H}= 7.30Hz), 5.90 (t (br), 1H, CO-NH), 5.00 (d, 1H, O-CH₂-O, ²J_{H.H}= 7.30Hz), 4.90 (d, 1H, O-CH₂-O, ²J_{H.H}= 7.30Hz), 4.35 (d, 2H, CH-CH₂), ³J_{H.H}= 6.67 Hz), 4.28 (q, 1H, CH₃-CHOH, ³J_{H.H}= 6.67 Hz), 6.23 (t, 1H, CH-CH₂, ³J_{H.H}=6.67 Hz), 3.58 (d, 2H, NH-CH-CO, ³J_{H.H}= 7.1 Hz), 3.45 (s, 3H, CH₃O), 1.35 (d, 3H, CH₃-CHOH, ³J_{H.H}= 6.67 Hz).- ¹³C-NMR (75 MHz, CDCl₃) : δ = 176.2 (s, COOH), 168.9 (s, CONH), 154.7 (s, NH-COO), 143.7 (s, Fluorenyl-C₆), 141.4 (s, Fluorenyl-C₅), 128.2 (d, Fluorenyl-C₁), 127.3 (d, Fluorenyl-C₄), 124.7 (d, Fluorenyl-C₃), 120.3 (d, Fluorenyl-H₂), 77.4 (d, NH-CHOH), 77.0 (t, O-CH₂-O), 68.1 (t, CH-CH₂), 67.4 (d, CH₃-CHOH), 62.2 (t, NH-CH-CO), 51.6 (q, CH₃O), 46.8 (d, CH-CH₂), 20.4 (q, CH₃-CHOH).- MS (FAB, 3-NBA) : m/z = 271 (5, [M+H]^{⊕}).

### Anwendung

Das geschützte Peptid (**LA.3.1**) wird in Lösung zusätzlich mit 20% Piperidin/DMF über 5h bei RT behandelt. Es ist keine Veränderung des Eduktes (HPLC-Analyse) zu erkennen. Behandlung mit 95% TFA/2.5% TIBS/2.5% Wasser führt zur simultanen Entschützung von BOC im Lysylrest und der Hydroxylfunktion des N.O-Acetals. Dieses entschützte Peptid zerfällt in gewünschter Weise in das Peptidamid durch Behandlung mit Puffersystem (a) bis (g). Die Reaktion verläuft innerhalb 15min bei 50°C.

### Syntheseweg B / Beispiel 4

### H-Lys(Boc)-Phe-Phe-α-rac-(SEM)oxyglycyl-βAla-OH (LB.4.1)

### Struktur

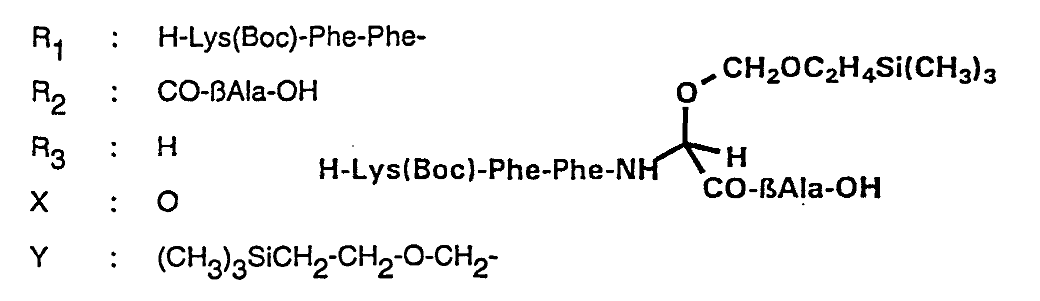

### Syntheseweg

### N^{α}-9-Fmoc-L-Phe-rac-α-(SEM)oxy-glycinbenzylester (LB.4.2)

### Summenformel (C₃₉H₄₄N₂O₇S)

Analog zu (**LA.3.5**) wird (**LB.4.2**) in DMF durch Reaktion mit einem Überschuß von 2 eq. Trimethylsilylethoxymethylchlorid synthetisiert. Die Isolierung erfolgt RP-C₁₈-HPLC chromatograpisch isoliert.- Ausbeute : 60.45 mg (70% d. Th.).-¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 14H, Fluorenyl-H /Phenyl-H), 6.1 (d, 1H, NH), 5.47 (d, 1H, NH-CH-O), 5.24 ('d', 2H, COOCH₂), 4.94 (d, 1H, O-CH₂-O, ²J_{H.H}= 7.20Hz), 4.75 (d, 1H, O-CH₂-O, ²J_{H.H}= 7.20Hz), 4.40-4.27 (m, 3H, NH-CH-CO / CH-CH₂), 4.15 (t, 1H, CH-CH₂, ³J_{H.H}=6.70Hz), 3.82 (AB-t, 4H, CH₂-CH₂), 3.0 (m, 1H, CH₂-C₆H₅), 0.1 (s, 3H, Si(CH₃)₃)..- ¹³C-NMR (75 MHz, CDCl₃) : δ = 174.4 (s, COO), 172.1 (s, CO-NH₂), 156.4 (s, NH-COO), 143.7 (s, Fluorenyl-C₆), 141.4 (s, Fluorenyl-C₅), 137.2 (s, Phenyl-H), 134.4 (s, Phenyl-H), 130.0-120.5 (8 Signale) (d, Fluorenyl-H / Phenyl-H), 77.0 (t, O-CH₂-O), 72.1 (d, NH-CHO), 68.1 (t, CH-CH₂), 67.4 (t, CH₂-CH₂), 67.2 (t, COO-CH₂), 56.6/55.4^{[dia]} (d, NH-CH-CO), 47.3 (d, CH-CH₂), 37.9 (t, CH₂-C₆H₅), 2.0 (q, Si(CH₃)₃).

### N^{α}-9-Fmoc-L-phenylalanyl-α-(trimethylsilylethoxymethyl)oxy-glycin (LB.4.3)^{(AB)}

### Summenformel (C₂₁H₂₃NO₄S)

Analog (**LA.1.5**) wird (**LB.4.3**) aus (**LB.4.2**) synthetisiert.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 14H, Fluorenyl-H / Phenyl-H), 6.1 (d, 1H, NH), 5.47 (d, 1H, NH-CH-O), 4.94 (d, 1H, O-CH₂-O, ²J_{H,H}= 7.20Hz), 4.75 (d, 1H, O-CH₂-O, ²J_{H.H}= 7.20Hz), 4.40-4.27 (m, 3H, NH-CH-CO / CH-CH₂), 4.15 (t, 1H, CH-CH₂, ³J_{H.H}=6.70Hz), 3.82 (AB-t, 4H, CH₂-CH₂), 3.0 (m, 1H, CH₂-C₆H₅), 0.1 (s, 3H, Si(CH₃)₃)..- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 134.4 (s, Phenyl-H), 130.0-120.5 (8 Signale) (d, Fluorenyl-H / Phenyl-H), 77.0 (t, O-CH₂-O), 72.1 (d, NH-CHO), 68.1 (t, CH-CH₂), 67.4 (t, CH₂-CH₂), 56.6/55.4^{[dia]} (d, NH-CH-CO), 47.3 (d, CH-CH₂), 37.9 (t, CH₂-C₆H₅), 2.0 (q, Si(CH₃)₃)..- MS (FAB, Thioglycerin) : m/z = 461 (15, [M+H]^{⊕}).

### H-Lys(Boc)-Phe-Phe-α-rac-(SEM)oxyglycyl-βalanin (LB.4.1)^{(MV)}

### Summenformel (C₂₁H₂₃NO₄S)

Nach allgemeinen Peptidsynthesemethoden wird (**LB.4.1**) an einem o-Chlorotrityl-funktionalisierten Harz aufgebaut und als geschütztes Peptid nach bekannten Verfahren abgelöst.- MS (FAB) : M/Z (3-NBA) = 157 ([M+H]^{⊕}).

### Anwendung

Die Behandlung mit 95% TFA/2.5% TIBS/2.5% Wasser führt zur simultanen Entschützung von BOC im Lysylrest und der Hydroxylfunktion des N.O-Acetals. Dieses entschützte Peptid zerfällt in gewünschter Weise in das Peptidamid durch Behandlung mit Puffersystem (a) bis (g). Die Reaktion verläuft innerhalb 15min bei 50°C.

Zusätzlich zum geschützten Peptid (**LB.4.1**) wird eine Modellverbindung (LB.4.4) am o-Chlorotrityl-funktionalisierten Polystyrolharz synthetisiert und als geschütztes Peptid vom Harz abgespalten. Dieses geschützte Peptid wird mit 0.2 M Tetrabutylammoniumfluorid/Acetonitril über 5h behandelt. Dadurch wird die Hydroxylfunktion des N.O-Acetals selektiv entschützt. Die Behandlung mit Puffersystem (g) im Gemisch mit 35% Ethanol führt zum geschützten Peptidamid H-Lys(Boc)-Trp(Boc)-Asp(tBu)-Asn(Trt)-Phe-NH₂.

### H-K(Boc)-W(Boc)-D(tBu)-N(Trt)-F-α-rac-(SEM)oxyglycyl-βAla-OH (LB.4.4)

### Summenformel (C₂₁H₂₃NO₄S)

MS (FAB) : M/Z (3-NBA) = 1557 ([M+H]^{⊕}).

### Syntheseweg B / Beispiel 5

### H-Lys(Boc)-Phe-Phe-α-rac-tert-butoxy-glycyl-βAla-OH (LB.5.1)

### Struktur

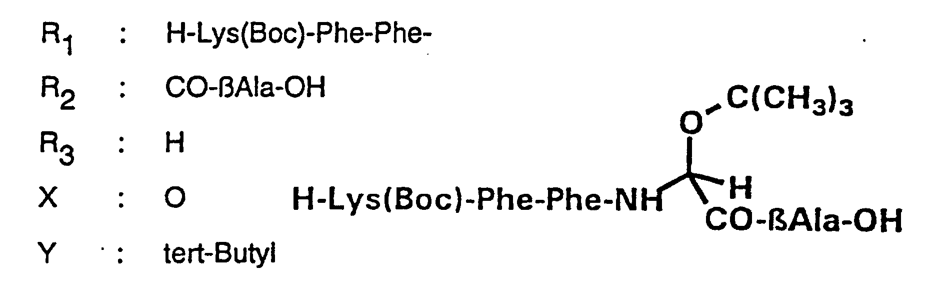

### Syntheseweg

### N^{α}-9-Fmoc-phenylalanyl-α-rac-tert-butoxy-glycinbenzylester (LB.5.2)

### Summenformel (C₂₈H₂₉NO₅)

110 mg (25 10⁻⁵ mol) (**LB.2.4**) werden unter Rückfluß in 2 ml absoluten THF mit 55 µl dest. (75 10⁻⁵ mol) Thionylchlorid über 1h umgesetzt. Das Reaktionsgemisch wird vollständig eingeengt und kurz im HV behandelt. Es werden 2 ml absoluter tert-Butanol und 42 µl (25 10⁻⁵ mol) Ethyldiisopropylamin zugesetzt und 2h refluxiert. Das Reaktionsgemisch wird in eine gesättigte wässrige NaCl-Lösung gegossen und die wässrige Phase 2x mit 100ml Ethylacetat extrahiert. Die organische Phase wird über MgSO₄ getrocknet und eingeengt. (**LB.5.2**) wird entweder RP-C₁₈-HPLC chromatograpisch zur Homogenität aufgereinigt.- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 14H, Fluorenyl-H / Phenyl-H), 6.1 (d, 1H, NH), 5.47 (d, 1H, NH-CH-OH), 5.23 ('d', 2H COOCH₂), 4.40-4.27 (m, 3H, NH-CH-CO / CH-CH₂), 4.15 (t, 1H, CH-CH₂, ³J_{H.H}=6.70Hz), 3.0 (m, 2H, CH₂-C₆H₅), 1.25 (s, 9H, C(CH₃)₃).- ¹³C-NMR (75 MHz, CDCl₃) : δ = 174.4 (s, COO), 172.1 (s, CO-NH₂), 156.4 (s, NH-COO), 143.7 (s, Fluorenyl-C₆), 141.4 (s, Fluorenyl-C₅), 137.2 (s, Phenyl-H), 135.2 (s, Phenyl-H), 130.0-120.5 (8 Signale/z.T aufgespalten^{[dia]}) (d, Fluorenyl-H / Phenyl-H), 74.6 (s, C(CH₃)₃), 72.1 (d, NH-CHOH), 68.1 (t, CH-CH₂), 67.5 (t, COO-CH₂), 56.6/55.4^{[dia]} (d, NH-CH-CO), 47.3 (d, CH-CH₂), 37.9 (t, CH₂-C₆H₅), 28.2 (q , C(CH₃)₃).- MS (FAB, Thioglycerin) : m/z = 461 (15, [M+H]^{⊕}).

### N^{α}-9-Fmoc-phenylalanyl-α-rac-tert-butoxy-glycin (LB.5.3)^{(AB)}

### Summenformel (C₁₉H₂₃NO₅)

115 mg (25 10⁻⁵ mol) (**LB.5.2**) werden in 3ml abs. Ethanol/Ethylacetat (1:2) gelöst. Es wird eine Spatelspitze Palladium/Aktivkohle (Fluka) zugegeben und 25 min Wasserstoff durch die Lösung geleitet. Der Katalysator wird abfiltriert und (**LB.5.3**) RP-C₁₈-HPLC chromatograpisch isoliert.- Ausbeute : 60.45 mg (70 % d. Th.).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 9H, Fluorenyl-H / Phenyl-H), 6.1 (d, 1H, NH), 5.47 (d, 1H, NH-CH-OH), 4.40-4.27 (m, 3H, NH-CH-CO / CH-CH₂), 4.15 (t, 1H, CH-CH₂, ³J_{H.H}=6.70Hz), 3.0 (m, 2H, CH₂-C₆H₅), 1.25 (s, 9H, C(CH₃)₃).- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 135.2 (s, Phenyl-H), 130.0-120.5 (5 Signale/z.T aufgespalten^{[dia]}) (d, Fluorenyl-H / Phenyl-H), 74.6 (s, C(CH₃)₃), 72.1 (d, NH-CHOH), 68.1 (t, CH-CH₂), 56.6/55.4^{[dia]} (d, NH-CH-CO), 47.3 (d, CH-CH₂), 28.2 (q , C(CH₃)₃).

### H-Lys(Boc)-Phe-Phe-α-rac-tert-butoxy-glycyl-βAla (LB.5.1)^{(MV)}

### Summenformel (C₃₈H₄₆N₆O₉)

Das geschützte Peptid (**LB.5.1**) wird nach üblichen Bedingungen an einem o-Chlorotrityl-funktionalisierten Harz unter Verwendung von (**LB.5.2**) aufgebaut und vom Träger abgespalten. Die Freisetzung des NH₂.OR-Acetals erfolgt mit 20% Piperidin/ DMF.- MS (FAB, Thioglycerin) : m/z = 731 (15, [M+H]^{⊕}).

### Anwendung

Das geschützte Peptid (**LB.5.1**) zeigt vollständige Stabilität gegenüber 20% Piperidin/DMF (gezeigt durch durch quntitative UV/VIS-Analytik der einzelnen Kopplungsschritte und durch Behandlung des geschützten Peptides (**LB.5.1**) in Lösung mit dem oben genannten Reagenz). Nach Abspaltung der Hydroxylschutzgruppe nach üblichen Verfahren (und simultan von Boc im Lysylrest) wird das entschützte Peptid mit Puffersystem (a), (b) und (g) behandelt. In gewünschter Weise zerfällt die Modellverbindung (**LB.5.1**) in das Peptidamid H-Lys-Phe-Phe-NH₂.

### Syntheseweg B / Beispiel 6

### H-Lys(Boc)-Phe-Phe-α-rac-methoxyglycyl-βAla-OH (LB.6.1)

### Struktur

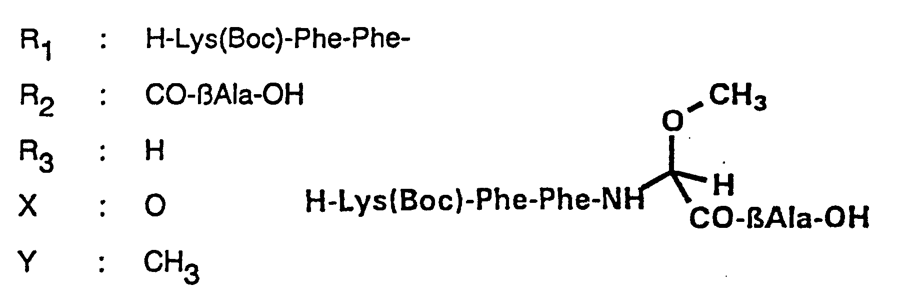

### Syntheseweg

### N^{α}-9-Fmoc-L-phenylalanyl-rac-α-methoxy-glycinmethylester (LB.6.2)

### Summenformel (C₂₇H₂₈N₂O₆)

Analog zu (**PA.1.3**) wurde durch säurekatalysierte Reaktion aus (**LB.2.4**) in Methanol (**LB.6.2**) synthetisiert.- Ausbeute : (95% d. Theorie).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 9H, Fluorenyl-H / Phenyl-H), 6.1 (d, 1H, NH), 5.47 (d, 1H, NH-CH-OH), 4.40-4.27 (m, 3H, NH-CH-CO / CH-CH₂), 4.23 (t, 1H, CH-CH₂, ³J_{H.H}=6.67 Hz), 3.81 (s, 3H, CH₃), 3.42 (s, 3H, CH₃), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz).- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 130.0-120.5 (5 Signale) (d, Fluorenyl-H / Phenyl-H), 72.1 (d, NH-CHOH), 68.1 (t, CH-CH₂), 56.6/55.4^{[dia]} (d, NH-CH-CO), 53,0/52.4 (q, CH₃), 47.3 (d, CH-CH₂), 37.9 (t, CH₂-C₆H₅).- MS (FAB, Thioglycerin) : m/z = 477 (17, [M+H]^{⊕}).

### N^{α}-9-Fmoc-L-phenylalanyl-α-methoxy-glycin (LB.6.3)^{(AB)}

### Summenformel (C₂₀H₂₁N₂O₆)

Analog zu (**LA.2.4**) wurde die Carboxylfunktion von (**LB.6.2**) in Aceton/Wasser unter Katalyse von LiOH freigesetzt.- Ausbeute : (62% d. Theorie).- ¹H-NMR (400 MHz, CDCl₃) : δ = 7.81 (d, 2H, Fluorenyl-H¹, ³J_{H.H}= 7.30 Hz), 7.67 (d, 2H, Fluorenyl-H⁴, ³J_{H.H}=7.26 Hz), 7.40-7.15 (m, 9H, Fluorenyl-H / Phenyl-H), 6.1 (d, 1H, NH), 5.47 (d, 1H, NH-CH-OH), 4.40-4.27 (m, 3H, NH-CH-CO / CH-CH₂), 4.23 (t, 1H, CH-CH₂, ³J_{H.H}=6.67 Hz), 3.81 (s, 3H, CH₃), 3.42 (s, 3H, CH₃), 3.10 (AB-q, 1H, CH₂-C₆H₅, ³J_{H.H}= 6.7Hz, ²J_{H.H}= 14.0Hz), 2.95 (AB-q, 1H, CH₂-C₆H₅, ³J_{H.H}= 6.7Hz, ²J_{H.H}= 14.0Hz).- ¹³C-NMR (75 MHz, CDCl₃) : δ = 174.4 (s, COOH), 172.1 (s, CO-NH₂), 156.4 (s, NH-COO), 143.7 (s, Fluorenyl-C₆), 141.4 (s, Fluorenyl-C₅), 137.2 (s, Phenyl-H), 130.0-120.5 (5 Signale) (d, Fluorenyl-H / Phenyl-H), 72.1 (d, NH-CHOH), 68.1 (t, CH-CH₂), 56.6/55.4^{[dia]} (d, NH-CH-CO), 53,0 (q, CH₃), 47.3 (d, CH-CH₂), 37.9 (t, CH₂-C₆H₅).- MS (FAB, Thioglycerin) : m/z = 385 (10, [M+H]^{⊕}).

### Syntheseweg A / Syntheseweg B / Beispiel 7

### Übertragung auf die routinemäßige Peptidsynthese

Zusätzlich zu den Untersuchungen in Lösung und der Synthese der oben aufgeführten Modellverbindungen wurden mit den Linkerbausteinen (**LA.6.1**), (**LA.7.2**) und (**LB.3.2**) Peptide unterschiedlicher Sequenz (Sequenzlänge bis 10 Aminosäurereste) unter Verwendung eines aminofunktionalisierten Polyethylenglycolharzes (TentaGel S Amine) bzw. auf β-Alanin-funktionalisierten Cellulosepapier (Whatman 3MM) aufgebaut und im Falle der Verwendung von (**LB.3.2**) mit 95 % TFA/2.5 % TIBS/2.5 % Wasser und im Falle der Verwendung von (**LA.6.1**) und (**LA.7.2**) in dem oben beschriebenen zweistufigen Verfahren entschützt. Die Polymermaterialien werden daraufhin je dreimal 10 min mit MeOH/Wasser 1:1 (0.1 % HCl) und 1 M Essigsäure/Wasser gewaschen und im HV 12 h getrocknet. Die Abspaltung der Peptidamide erfolgt in Puffersystem (b) (siehe unten) bei 50°C und führt zu Peptidamiden mit erwarteter Reinheit.

Die Ergebnisse zeigen deutlich,
- daß das als Schutzgruppe bzw. Ankergruppe eingesetzte und entsprechend geschützte N.O-/N.S-Acetale unter den basischen Reaktionsbedingungen (z.B. 20% Piperidin in DMF) der Synthese von R₁ stabil ist (Fmoc SPPS).
- daß das als Ankergruppe eingesetzte und entsprechend geschützte N.S-Acetal unter den aciden Reaktionsbedingungen (z.B. 55% TFA/DCM) der Synthese von R₁ stabil ist (Boc SPPS).
- daß das entschützte N.O-/N.S-Acetal unter den sauren wäßrigen Bedingungen stabil ist und die entsprechend geschützten Verbindungen gereinigt werden können.
- daß eine Abspaltung der Schutzgruppe (mit entschützter Hydroxyl- bzw. Thiolfunktion) unter neutralen Reaktionsbedingungen (pH = 7) möglich ist.
- das das Konzept sowohl als Schutzgruppe als auch als Ankergruppe verwendet werden kann.

### Verwendete Puffer

(a) NaH₂PO₄/Na₂HPO₄/0.1M/pH 7.0/H₂O
(b) NaH₂PO₄/Na₂HPO₄/0.1M/pH 7.5/H₂O
(c) NaH₂PO₄/Na₂HPO₄/0.01M/pH 7.0/H₂O
(d) NaH₂PO₄/Na₂HPO₄/0.01M/pH 7.5/H₂O
(e) tris-Hydroxymethylaminomethan-hydrochlorid (Tris.HCl)/0.01 M/pH 7.6/H₂O
(f) tris-Hydroxymethylaminomethan-hydrochlorid (Tris.HCl)/0.01 M/pH 8.0/H₂O
(g) Triethylammoniumacetat (TEAAc)/0.01 M/pH 7.0/H₂O

### Verwendete Abkürzungen

Aminosäurederivate nach IUPAC-IUB [J. Biol. Chem. 260, 14(1983)]
- Boc: tert.-Butyloxycarbonyl
- tBu: tert-Butyl
- DCHA: Dicyclohecylammonium
- DCM: Dichlormethan
- DIC: N,N'-Diisopropylcarbodiimid
- DMF: Dimethylformamid
- DMSO: Dimethylsulfoxid
- Et: Ethyl
- FAB-MS: "Fast Atom Bombardement" Massenspektroskopie
- Fmoc: 9-Fluorenylmethoxycarbonyl
- Hal: Halogen
- HOBt: N-Hydroxybenzotriazol
- HPLC: Hochdruckflüssigchromatographie
- HV: Hochvakuum
- Me: Methyl
- Melm: N-Methylimidazol
- MOM: Methoxymethyl
- MS: Massenspektroskopie
- MSNT: Mesitylensulfonyl-3-nitro-1.2.4-triazol
- 3-NBA: 3-Nitrobenzylalkohol
- NMR: Kernresonanzspektroskopie
- SEM: Trimethylsilylethoxymethyl
- TBDMS: t-Butyl-dimethylsilyl
- TIBS: Trüsobutylsilan
- TFA: Trifluoressigsäure
- Trt: Trityl

## Patentansprüche

1. Verfahren zur Herstellung eines durch eine temporäre Schutzgruppe geschützten Carbamids der allgemeinen Formel (I)
R₁-CO-NH-C(R₂)(R₃)-X-Y (I)
worin
R₁-CO- einen Rest eines Naturstoffs als Produkt einer mehrstufigen Synthese bedeutet;
R₂ und R₃ Reste der Carbamid-Schutzgruppe bedeuten, die nicht an deren Funktion teilhaben, wobei R₂ und R₃ (i) stark elektronenziehende Gruppen gemäß der Erlenmeyer-Regel für O.O-, N.O- oder N.S-Acetale bedeuten und gleich oder verschieden sein können, oder (ii) verschieden sind, wenn einer der beiden Reste ein Wasserstoffatom bedeutet und der andere der beiden Reste eine stark elektronenziehende Gruppe gemäß der Erlenmeyer-Regel für 0.0-, N.O- oder N.S-Acetale ist;
X ein Sauerstoffatom oder ein Schwefelatom bedeutet und
Y eine Schutzgruppe für X bei einer Safety-Catch-Gruppierung bedeutet,
**dadurch *gekennzeichnet,* daß** man
(i) eine Verbindung der Formel
H₂N-C(R₂)(R₃)-X-Y
mit einer Verbindung der Formel
R₁-COOH
kondensierend umsetzt, wobei R₁-CO-, R₂, R₃, X und Y die vorstehend angegebenen Bedeutungen besitzen, und gegebenenfalls von der gebildeten Verbindung der Formel (I)
(ii) die Schutzgruppe Y abspaltet und das gebildete Zwischenprodukt (Zwischenstufe) bei einem pH außerhalb des Bereichs von pH 5 bis 9 stabilisiert und
(iii) danach im pH-Bereich von 5 bis 9 C(R₂)(R₃)=X freisetzt und das gebildete Zwischenprodukt in das freie Carbamid überführt.

2. Verfahren zur Herstellung eines durch eine temporäre Schutzgruppe geschützten Carbamids der allgemeinen Formel (I)
R₁-CO-NH-C(R₂)(R₃)-X-Y (I)
worin
R₁-CO- einen Rest eines Naturstoffs als Produkt einer mehrstufigen Synthese bedeutet;
R₂ und R₃ Reste der Carbamid-Schutzgruppe bedeuten, die nicht an deren Funktion teilhaben, wobei R₂ und R₃ (i) stark elektronenziehende Gruppen gemäß der Erlenmeyer-Regel für O.O-, N.O- oder N.S-Acetale bedeuten und gleich oder verschieden sein können, oder (ii) verschieden sind, wenn einer der beiden Reste ein Wasserstoffatom bedeutet und der andere der beiden Reste eine stark elektronenziehende Gruppe gemäß der Erlenmeyer-Regel für 0.0-, N.O- oder N.S-Acetale ist;
X ein Sauerstoffatom oder ein Schwefelatom bedeutet und
Y eine Schutzgruppe für X bei einer Safety-Catch-Gruppierung bedeutet, **dadurch *gekennzeichnet,* daß** man
(i)
(a) eine Verbindung der Formel C(R₂)(R₃)=X mit einer Verbindung der Formel R₁-CO-NH₂ zu einer Verbindung der Formel R₁-CO-NH-C(R₂)(R₃)-XH umsetzt und
(b) die -XH-Gruppe des Reaktionsprodukts gemäß (a) in eine -X-Y-Gruppe überführt,
wobei R₁, R₂, R₃, X und Y die vorstehend angegebenen Bedeutungen besitzen, und gegebenenfalls von der gebildeten Verbindung der Formel (I)
(ii) die Schutzgruppe Y abspaltet und das gebildete Zwischenprodukt (Zwischenstufe) bei einem pH außerhalb des Bereichs von pH 5 bis 9 stabilisiert und
(iii) danach im pH-Bereich von 5 bis 9 C(R₂)(R₃)=X freisetzt und das gebildete Zwischenprodukt in das freie Carbamid überführt.

3. Verfahren nach Anspruch 1 oder 2, **dadurch *gekennzeichnet,* daß** R₁-CO- eine Einheit für die Kette eines Peptids mit ein oder mehreren Aminosäureresten oder einen Carbonylrest eines Produkts einer mehrstufigen Peptidsynthese bedeutet.

4. Verfahren nach einem der vorhergehenden Ansprüche, **dadurch *gekennzeichnet*, daß** R₂ und/oder R₃ eine Halogenalkylgruppe, insbesondere eine Trifluormethylgruppe, oder eine ggf. derivatisierte Carboxylgruppe, insbesondere eine -CO-βAla-OH-Gruppe, oder eine Alkylestercarboxylgruppe bedeuten, insbesondere eine - COOCH₃-Gruppe.

5. Verfahren nach einem der vorhergehenden Ansprüche, **dadurch *gekennzeichnet,* daß** R₂ oder R₃ eine zusätzliche reaktive Funktion zur Verknüpfung mit einem Trägermaterial aufweisen, insbesondere eine Carboxyl-, eine Amino- oder eine Thiolgruppe.

6. Verfahren nach einem der vorhergehenden Ansprüche, **dadurch *gekennzeichnet,* daß** die Schutzgruppe Y eine Alkylgruppe, insbesondere eine Methyl-, Ethyl-, i-Propyl- oder t-Butylgruppe, eine substituierte Alkylgruppe, insbesondere eine CH₃-O-CH₂-oder (CH₃)₃Si-CH₂-CH₂-O-CH₂-Gruppe, eine Arylgruppe oder eine Alkylsilylgruppe bedeutet, insbesondere eine t-Butyldimethylsilylgruppe.

7. Verfahren nach einem der vorhergehenden Ansprüche, **dadurch *gekennzeichnet,* daß** Y = CH₃ und R₂ = R₃ = CF₃ bedeuten.

8. Verfahren nach einem der vorhergehenden Ansprüche; **dadurch *gekennzeichnet,* daß** man nach Stufe (i) und vor Stufe (ii) eine Peptidsynthese durchführt.

9. Verfahren nach Anspruch 8, **dadurch *gekennzeichnet,* daß** man die Peptidsynthese an einem Trägermaterial durchführt.

10. Geschütztes Carbamid, erhältlich nach dem Verfahren gemäß Anspruch 5, **dadurch *gekennzeichnet,* daß** es mit einem Trägermaterial verknüpft ist.

## Claims

1. Process for producing a carbamide, protected by a temporary protective group, having the general formula (I)
R₁-CO-NH-C(R₂)(R₃)-X-Y (I)
wherein
R₁-CO- means a residue of a natural product as a product of a multi-stage synthesis;
R₂ and R₃ mean residues of the carbamide protective group which do not participate in their function, whereby R₂ and R₃ (i) can be identical or different and mean intensely electron-withdrawing groups according to the Erlenmeyer Rule for O.O, N.O or N.S acetals, or (ii) are different when one of the two residues means a hydrogen atom and the other of the two residues is an intensely electron-withdrawing group according to the Erlenmeyer Rule for O.O, N.O or N.S acetals;
X means an oxygen atom or a sulphur atom, and
Y means a protective group for X in a safety-catch grouping,
**characterised in that**
(i) a compound of the formula
H₂N-C(R₂) (R₃) -X-Y
is condensingly reacted with a compound of the formula
R₁-COOH
R₁-CO-, R₂, R₃, X and Y having the meanings given above, and if necessary of the formed compound of formula (I)
(ii) the protective group Y is split off and the formed intermediate product (intermediate stage) is stabilised at a pH beyond the range of pH 5 to 9 and
(iii) thereafter, in the range of pH of from 5 to 9 C(R₂)(R₃)=X is released and the formed intermediate product is converted into the free carbamide.

2. Process for producing a carbamide, protected by a temporary protective group, having the general formula (I)
R₁-CO-NH-C(R₂)(R₃)-X-Y (I)
wherein
R₁-CO- means a residue of a natural product as a product of a multi-stage synthesis;
R₂ and R₃ mean residues of the carbamide protective group which do not participate in their function, whereby R₂ and R₃ (i) can be identical or different and mean intensely electron-withdrawing groups according to the Erlenmeyer Rule for O.O, N.O or N.S acetals, or (ii) are different when one of the two residues means a hydrogen atom and the other of the two residues is an intensely electron-withdrawing group according to the Erlenmeyer Rule for O.O, N.O or N.S acetals;
X means an oxygen atom or a sulphur atom, and
Y means a protective group for X in a safety-catch grouping,
**characterised in that**
(i)
(a) a compound of the formula C(R₂) (R₃)=X is reacted with a compound of the formula R₁-CO-NH₂ to yield a compound of the formula R₁-CO-NH-C(R₂) (R₃) -XH, and
(b) the group -XH of the reaction product according to (a) is converted into a group -X-Y,
R₁, R₂, R₃, X and Y having the meanings given above, and if necessary of the formed compound of formula (I)
(ii) the protective group Y is split off and the formed intermediate product (intermediate stage) is stabilised at a pH beyond the range of pH 5 to 9 and
(iii)thereafter, in the range of pH of from 5 to 9 C(R₂)(R₃)=X is released and the formed intermediate product is converted into the free carbamide.

3. Process according to claim 1 or 2, **characterised in that** R₁-CO- means a unit for a chain of a peptide having one or a plurality of amino acid residues, or a carbonyl residue of a product of a multi-stage peptide synthesis.

4. Process according to one of the preceding claims, **characterised in that** R₂ and/or R₃ mean a halogen alkyl group, particularly, a trifluoromethyl group, or a carboxyl group, if necessary derivatised, especially a -CO- ala-OH group, or an alkyl ester carboxyl group, especially a -COOCH₃ group.

5. Process according to one of the preceding claims, **characterised in that** R₂ or R₃ have an additional reactive function for linkage to a carrier material, especially a carboxyl, amino or a thiol group.

6. Process according to one of the preceding claims, **characterised in that** the protective group Y means an alkyl group, especially a methyl, ethyl, i-propyl, or t-butyl group, a substituted alkyl group, especially a CH₃-O-CH₂- or (CH₃)₃Si-CH₂-CH₂-O-CH₂- group, an aryl group or an alkyl silyl group, especially a t-butyl dimethylsilyl group.

7. Process according to one of the preceding claims, **characterised in that** Y = CH₃ and R₂ = R₃ = CF₃.

8. Process according to one of the preceding claims, **characterised in that** a peptide synthesis is carried out after stage (i) and previous to stage (ii).

9. Process according to claim 8, **characterised in that** the peptide synthesis is carried out using a carrier material.

10. A protected carbamide obtainable according to the process of claim 5, **characterised in that** it is linked to a carrier material.

## Revendications

1. Procédé de préparation d'un carbamide protégé par un groupe protecteur temporaire, de la formule générale (I)
R₁-CO-NH-C(R₂)(R₃)-X-Y (I)
dans laquelle
R₁-CO- représente un résidu d'une substance naturelle en tant que produit d'une synthèse en plusieurs étapes ;
R₂ et R₃ représentent des résidus ou un groupe protégeant le carbamide et ne participant à sa fonction, où R₂ et R₃ (i) représentent des groupes fortement accepteurs d'électrons suivant la règle d'Erlenmeyer pour des acétals O.O, N.O ou N.S et peuvent être identiques ou différents, ou bien (ii) sont différents lorsque l'un des résidus ou les deux représente(nt) un atome d'hydrogène et que l'autre résidu ou les deux est (sont) un groupe fortement accepteur d'électrons suivant la règle d'Erlenmeyer pour des acétals O.O, N.O ou N.S ;
X représente un atome d'oxygène ou un atome de soufre, et
Y représente un groupe protecteur pour X dans le cas d'un groupe de blocage (safety catch grouping),
**caractérisé en ce que**
(i) on fait réagir un composé de la formule
H₂N-C(R₂)(R₃)-X-Y
avec un composé de la formule
R₁-COOH
en conditions de condensation, R₁-CO-, R₂, R₃, X et Y ayant les significations indiquées plus haut, et, le cas échéant,
(ii) on dissocie le groupe protecteur Y du composé de la formule (I) formé et on stabilise le produit intermédiaire (stade intermédiaire) formé à un pH en dehors de la plage de pH 5 à pH 9, et
(iii) on libère ensuite C(R₂) (R₃)=X dans la plage de pH 5 à pH 9 et on convertit le produit intermédiaire en carbamide libre.

2. Procédé de préparation d'un carbamide protégé par un groupe protecteur temporaire de la formule générale (X)
R₁-CO-NH-C(R₂)(R₃)-X-Y (I)
dans laquelle
R₁-CO- représente un résidu d'une substance naturelle en tant que produit d'une synthèse en plusieurs étapes ;
R₂ et R₃ représentent des résidus ou un groupe protégeant le carbamide et ne participant à sa fonction, où R₂ et R₃ (i) représentent des groupes fortement accepteurs d'électrons suivant la règle d'Erlenmeyer pour des acétals O.O, N.O ou N.S et peuvent être identiques ou différents, ou (ii) sont différents lorsque l'un des résidus ou les deux représente(nt) un atome d'hydrogène et que l'autre résidu ou les deux est/sont un groupe fortement accepteur d'électron suivant la règle d'Erlenmeyer pour des acétals O.O, N.O ou N.S ;
X représente un atome d'oxygène ou un atome de soufre, et
Y représente un groupe protecteur pour X dans le cas d'un groupe de blocage (safety catch grouping),
**caractérisé en ce que**
(i)
(a) on fait réagir un composé de la formule C(R₂)(R₃)=X avec un composé de la formule R₁-CO-NH₂ pour donner un composé de la formule R₁-CO-NH-C(R₂)(R₃)-XH, et
(b) on convertit le groupe -XH du produit réactionnel suivant (a) en un groupe -X-Y,
R₁, R₂, R₃, X et Y ayant les significations indiquées plus haut, et, le cas échéant,
(ii) on dissocie le groupe protecteur Y du composé formé de la formule (I) et on stabilise le produit intermédiaire (stade intermédiaire) à un pH en dehors de la plage de pH 5 à pH 9, et
(iii) on libère ensuite C(R₂)(R₃)=X dans la plage de pH 5 à pH 9 et on convertit le produit intermédiaire en carbamide libre.

3. Procédé selon la revendication 1 ou la revendication 2, **caractérisé en ce que** R₁-CO- représente un motif pour la chaîne d'un peptide avec un ou plusieurs résidus acides aminés ou un résidu carbonyle d'un produit d'une synthèse peptidique en plusieurs étapes.

4. Procédé selon l'une des revendications précédentes, **caractérisé en ce que** R₂ et/ou R₃ représentent un groupe haloalkyle, en particulier un groupe trifluorométhyle, ou un groupe carboxyle le cas échéant dérivatisé, en particulier un groupe -CO- Ala-OH, ou un groupe (ester alkylique)carboxyle, en particulier un groupe -COOCH₃.

5. Procédé selon l'une des revendications précédentes, **caractérisé en ce que** R₂ ou R₃ présentent en plus une fonction réactive pour la liaison à un matériau support, en particulier un groupe carboxyle, amino ou thiol.

6. Procédé selon l'une des revendications précédentes, **caractérisé en ce que** le groupe protecteur Y représente un groupe alkyle, en particulier un groupe méthyle, éthyle, isopropyle ou t-butyle, un groupe alkyle substitué, en particulier un groupe CH₃-O-CH₂- ou (CH₃)₃Si-CH₂-CH₂-O-CH₂-, un groupe aryle ou un groupe alkylsilyle, en particulier un groupe t-butyldiméthylsilyle.

7. Procédé selon l'une des revendications précédentes, **caractérisé en ce que** Y = CH₃ et R₂ = R₃ = CF₃.

8. Procédé selon l'une des revendications précédentes, **caractérisé en ce que**, après l'étape (i) et avant l'étape (ii), on effectue une synthèse peptidique.

9. Procédé selon la revendication 8, **caractérisé en ce que** l'on effectue la synthèse peptidique sur un matériau support.

10. Carbamide protégé pouvant être obtenu par le procédé selon la revendication 5, **caractérisé en ce qu'**il est lié à un matériau support.
